# DEMANDE DE BREVET EUROPEEN

(11) **EP 4 293 116 A2**
(43) Date de publication de la demande: **20.12.2023**
(21) Numéro de dépôt: 23184855.7
(22) Date de dépôt: 06.07.2017
(51) Int. Cl.: C12N 15/115

(54) **FIBRINOGÈNE LIQUIDE STABLE**

(30) Priorité: 06.07.2016 FR 1656479; 28.07.2016 EP 16305984; 28.07.2016 EP 16305985
(62) Demande divisionnaire de: 17742825.7
(71) Demandeur: Laboratoire Français du Fractionnement et des Biotechnologies, 92800 Puteaux (FR)
(72) Inventeur: BATAILLE, Damien, 91540 ORMOY (FR); TELLIER, Michel, 95600 EAUBONNE (FR)
(74) Mandataire: Plasseraud IP

(57) **Abrégé**

L'invention concerne une composition comprenant du fibrinogène stable sous forme liquide.

## Description

### Domaine de l'invention

La présente invention concerne une composition comprenant du fibrinogène stable sous forme liquide, utile en thérapie.

### Contexte de l'invention

Le fibrinogène est une protéine essentielle de la coagulation sanguine, car sa polymérisation en fibrine insoluble formée au terme de la cascade de réactions qui gouvernent la coagulation, aboutit à la formation d'un caillot obturant la brèche vasculaire, responsable du saignement.

La mise en place du caillot est ainsi essentielle pour assurer l'arrêt du saignement. De plus, la fibrine formée au niveau de la plaie constitue un réseau fibrillaire qui assure la réparation tissulaire, donc la cicatrisation.

De nombreuses pathologies sont actuellement traitées par voie intraveineuse en utilisant des compositions comprenant du fibrinogène. On peut citer par exemple les hypo-fibrinogénémies, dys-fibrinogénémies ou a-fibrinogénémies constitutionnelles chez les patients présentant une hémorragie spontanée ou post-traumatique, le traitement complémentaire dans la prise en charge d'une hémorragie sévère incontrôlée dans le cadre d'une hypofibrinogénémie acquise, etc.

Pour le traitement de certaines pathologies, l'utilisation de compositions comprenant du fibrinogène stables au stockage et prêtes à l'emploi peut s'avérer particulièrement avantageuse. Cette forme d'administration offre en effet plus de flexibilité et de rapidité d'administration aux praticiens, améliorant la prise en charge urgente des patients hémorragiques. A cette fin, des compositions comprenant du fibrinogène lyophilisées, stables au stockage et adaptées à une constitution rapide ont été développées. Cependant, la reconstitution de telles compositions lyophilisées nécessite quelques minutes. De plus, la reconstitution doit être réalisée doucement pour permettre la dissolution complète du lyophilisat, garantissant la concentration du produit, et ce sans formation de mousse, de trouble ou de dépôt qui rendrait la composition difficilement ou non administrable.

L'utilisation de tels produits lyophilisés n'est donc pas optimale dans un contexte de médecine d'urgence intra ou péri-hospitalière ou chaque minute compte pour le traitement de l'hémorragie.

A ce jour, les compositions comprenant du fibrinogène sont commercialisées sous forme lyophilisée à reconstituer et ne donnent pas entière satisfaction en termes de stabilité liquide notamment.

Notamment, Chabbat et al (Thrombosis Research, vol.76, No.6, 525-533, 1994), décrit une composition de fibrinogène comprenant du NaCl, de l'acide aminocaproïque, de la glycine, de l'arginine et de l'aprotinine. L'aprotinine, inhibiteur compétitif des sérines protéases naturel, est néanmoins défavorable aux patients lors de l'administration intraveineuse, d'une part du point de vue de son origine animale (préparation à partir de poumon de boeuf) avec un risque non négligeable de choc anaphylactique, et d'autre part vis-à-vis de son activité anticoagulante pouvant aggraver le défaut de coagulation du patient à traiter.

La demande WO0021568 décrit des compositions comprenant du fibrinogène, des ions calcium, un immuno-stimulateur, et des anti-fibrinolytiques, pour leur utilisation comme colle de fibrine. Néanmoins de telles compositions ne peuvent être administrées par voie intraveineuse aux patients.

La demande EP1648496 de la demanderesse, décrit quant à elle des compositions comprenant du fibrinogène, stabilisé par addition d'arginine, de citrate trisodique, de leucine/isoleucine et de glycine et/ou lysine, permettant la stabilisation et la solubilisation du lyophilisat. Une telle composition n'est en revanche pas totalement adaptée à une formulation liquide de fibrinogène.

Dans ce contexte, les besoins en compositions liquides comprenant du fibrinogène d'utilisation aisée persistent.

La Demanderesse a mis au point une nouvelle composition comprenant du fibrinogène, préférentiellement du fibrinogène humain, qui est stable à l'état liquide.

L'invention concerne donc une composition comprenant du fibrinogène stable sous forme liquide.

### Résumé de l'invention

De manière surprenante et avantageuse, la Demanderesse a mis en évidence qu'il est possible d'obtenir des compositions comprenant du fibrinogène particulièrement stables dans le temps sous forme liquide, en utilisant un nombre limité et des quantités minimales d'excipients.

### Brève description des figures

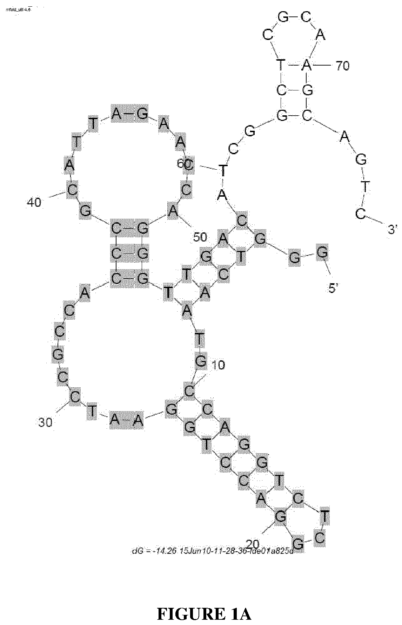

La **figure 1A** représente la structure secondaire prédite d'un aptamère de SEQ ID N° : 1. Les nucléotides appartenant à la séquence de base (SEQ ID N° : 66) sont surlignés en gris.

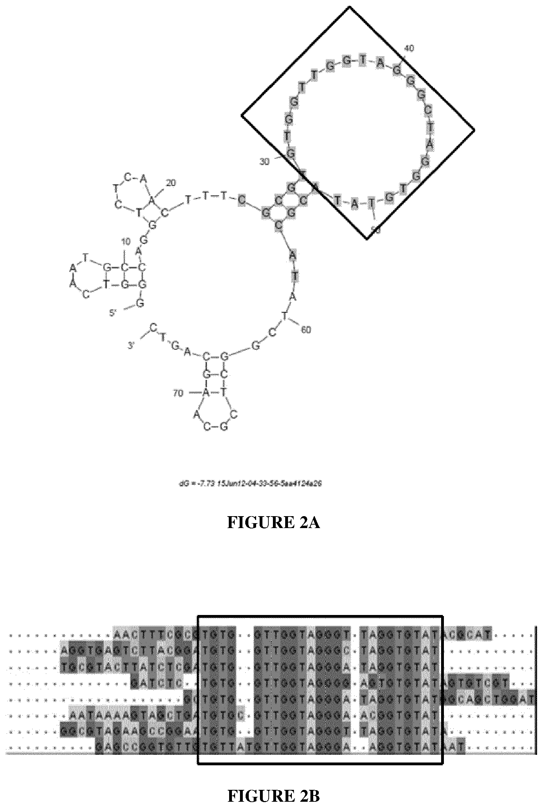

La **figure 2A** représente la structure secondaire prédite d'un aptamère de SEQ ID N° : 58. Les nucléotides appartenant à la séquence de base (SEQ ID N° : 67) sont surlignés en gris. La boucle encadrée correspond à la région de l'aptamère comprenant le groupement consensus de formule (III).
La **figure 2B** représente les alignements des séquences de base de SEQ ID N° : 67-74. Les parties encadrées des séquences comprennent le groupement consensus de formule (III).

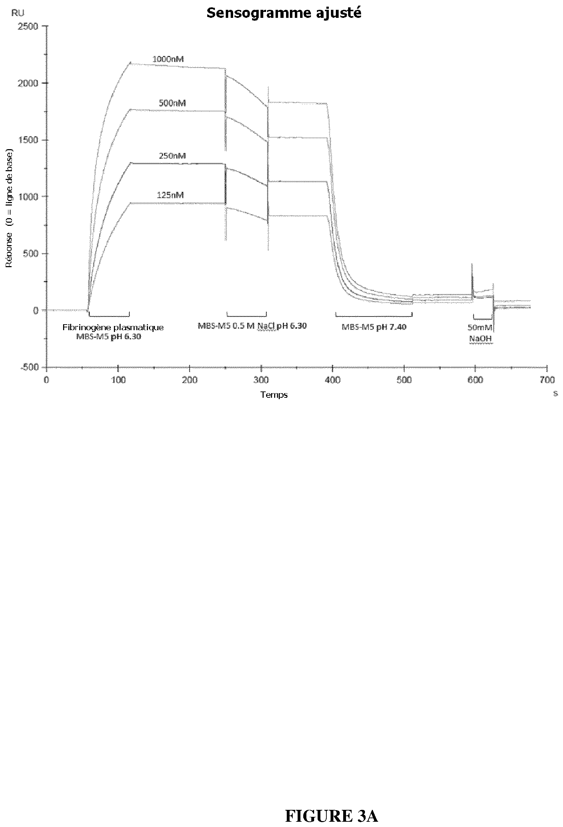

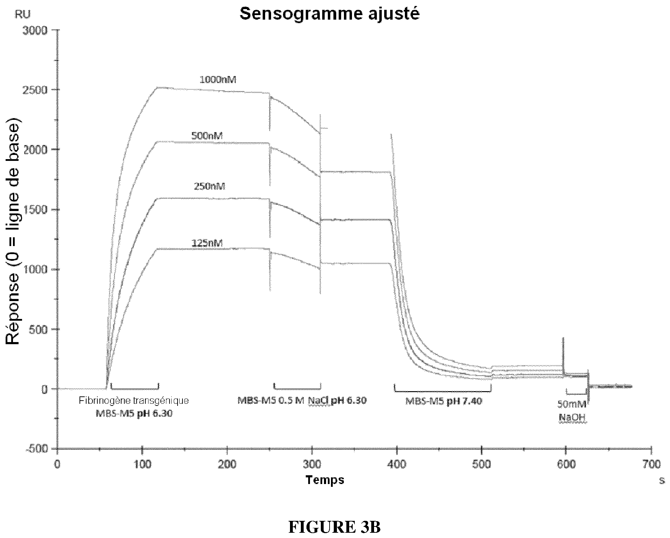

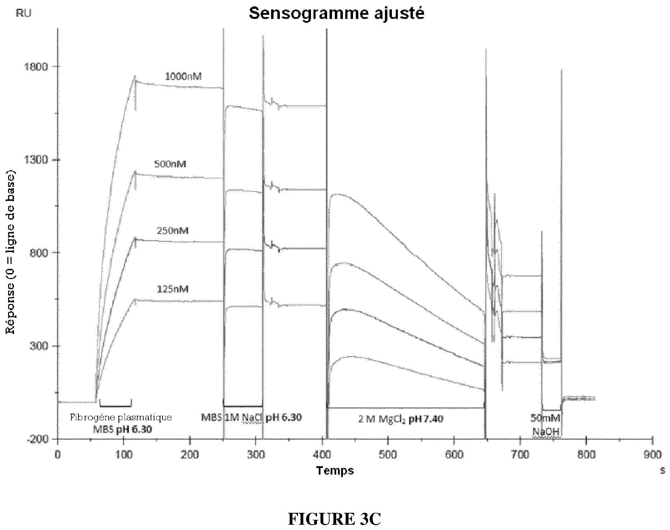

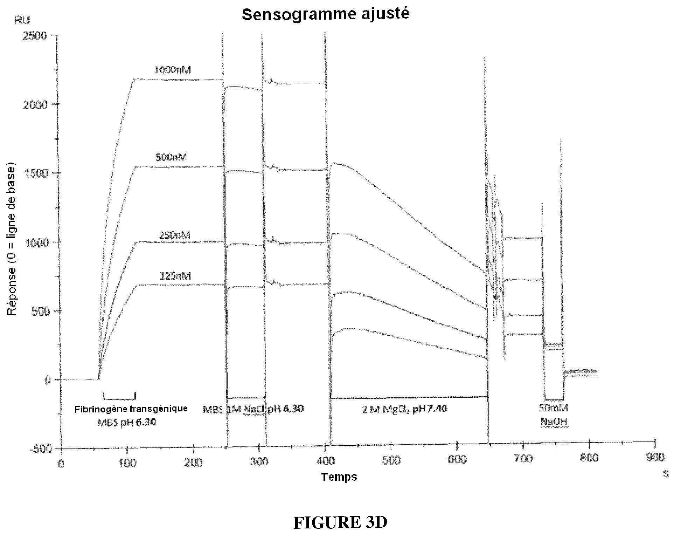

Les **figures 3-4** représentent les propriétés de liaison de certains aptamères dirigés contre un fibrinogène humain obtenu par le procédé de l'invention.
La **figure 3A** représente les courbes de liaison SPR (résonance plasmonique de surface) d'un fibrinogène plasmatique humain présent en une concentration de 125 nM à 1000 nM sur la SEQ ID N° : 66 (la séquence de base de SEQ ID N° : 1) immobilisé sur une puce. Chaque solution de fibrinogène plasmatique humain a été injectée à pH 6,3 de sorte qu'un complexe s'est formé d'une façon dose-dépendante comme le montre l'augmentation des signaux dépendant de la concentration de fibrinogène. L'injection d'une solution tampon à pH 6,3 comprenant 0,5 M de NaCl n'a pas significativement induit l'élution du fibrinogène plasmatique humain. Le fibrinogène a ensuite été libéré du complexe par un tampon d'élution à pH 7,40. Le support solide a ensuite été régénéré par injection d'une solution de NaOH 50 mM. Axe des X : temps en s. Axe des Y : réponse SPR en unité arbitraire.
La **figure 3B** représente les courbes de liaison SPR d'un fibrinogène transgénique présent en une concentration de 125 nM à 1000 nM sur la SEQ ID N° : 66 (la séquence de base de SEQ ID N° : 1) immobilisé sur une puce. Chaque solution de fibrinogène transgénique a été injectée à pH 6,3 de sorte qu'un complexe s'est formé d'une façon dose dépendante comme le montre l'augmentation des signaux dépendant de la concentration de fibrinogène. L'injection d'une solution tampon à pH 6,3 comprenant du NaCl 0,5 M n'a pas significativement induit l'élution du fibrinogène transgénique. Le fibrinogène a ensuite été libéré du complexe par un tampon d'élution à pH 7,40. Le support solide a ensuite été régénéré par injection d'une solution de NaOH 50 mM. Axe des X : temps en s. Axe des Y : réponse SPR en unité arbitraire.
La **figure 3C** représente les courbes de liaison SPR d'un fibrinogène plasmatique humain présent en une concentration de 125 nM à 1000 nM sur la SEQ ID N° : 67 (la séquence de base de SEQ ID N° : 58) immobilisé sur une puce. Chaque solution de fibrinogène plasmatique humain a été injectée à pH 6,3 de sorte qu'un complexe s'est formé d'une façon dose dépendante comme le montre l'augmentation des signaux dépendant de la concentration de fibrinogène. L'injection d'une solution tampon à pH 6,3 comprenant du NaCl 1M n'a pas significativement induit l'élution du fibrinogène plasmatique humain. Le fibrinogène a ensuite été libéré du complexe par un tampon d'élution à pH 7,40 et contenant du MgCl₂ 2M. Le support solide a ensuite été régénéré par injection d'une solution de NaOH 50 mM. Axe des X : temps en s. Axe des Y : réponse SPR en unité arbitraire.
La **figure 3D** représente les courbes de liaison SPR d'un fibrinogène transgénique présent en une concentration de 125 nM à 1000 nM sur la SEQ ID N° : 67 (la séquence de base de SEQ ID N° : 58) immobilisé sur une puce. Chaque solution de fibrinogène transgénique a été injectée à pH 6,3 de sorte qu'un complexe s'est formé d'une façon dose dépendante comme le montre l'augmentation des signaux dépendant de la concentration de fibrinogène. L'injection d'une solution tampon à pH 6,3 comprenant du NaCl 1M n'a pas considérablement induit l'élution du fibrinogène plasmatique humain. Le fibrinogène a ensuite été libéré du complexe par un tampon d'élution à pH 7,40 et contenant du MgCl₂ 2M. Le support solide a ensuite été régénéré par injection d'une solution de NaOH 50 mM. Axe des X : temps en s. Axe des Y : réponse SPR en unité arbitraire.

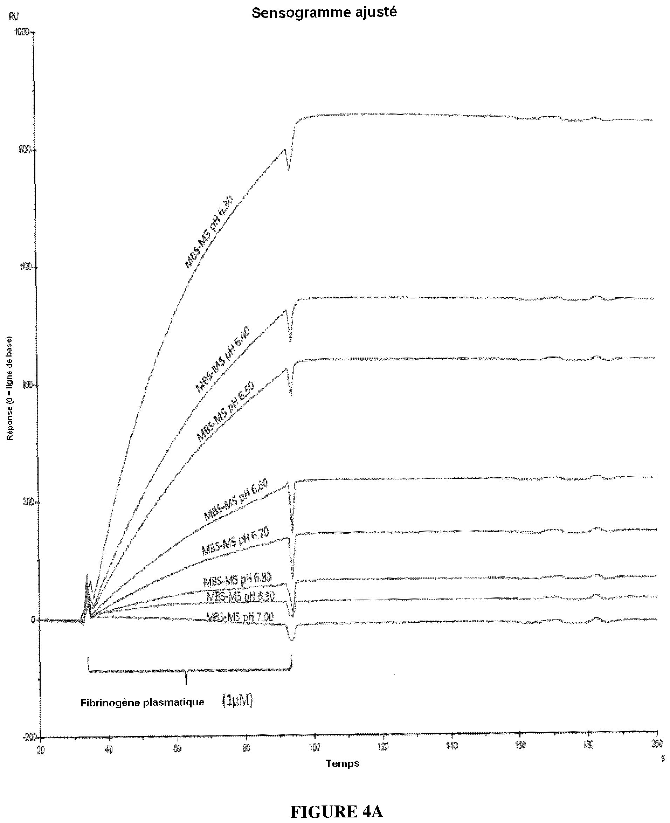

La **figure 4A** représente des sensogrammes SPR illustrant la dépendance de la liaison du fibrinogène à l'aptamère immobilisé de SEQ ID N° : 66 (la séquence de base de SEQ ID N° : 1) vis-à-vis du pH. Le fibrinogène plasmatique est injecté à différents pH, après l'injection d'un échantillon, un tampon de migration à pH 6,30 est envoyé dans la cellule d'écoulement à chaque essai. Le niveau de liaison le plus élevé est obtenu à un pH de 6,30. Le niveau de liaison diminue lorsque le pH augmente. Axe des X : temps en s. Axe des Y : réponse SPR en unité arbitraire.

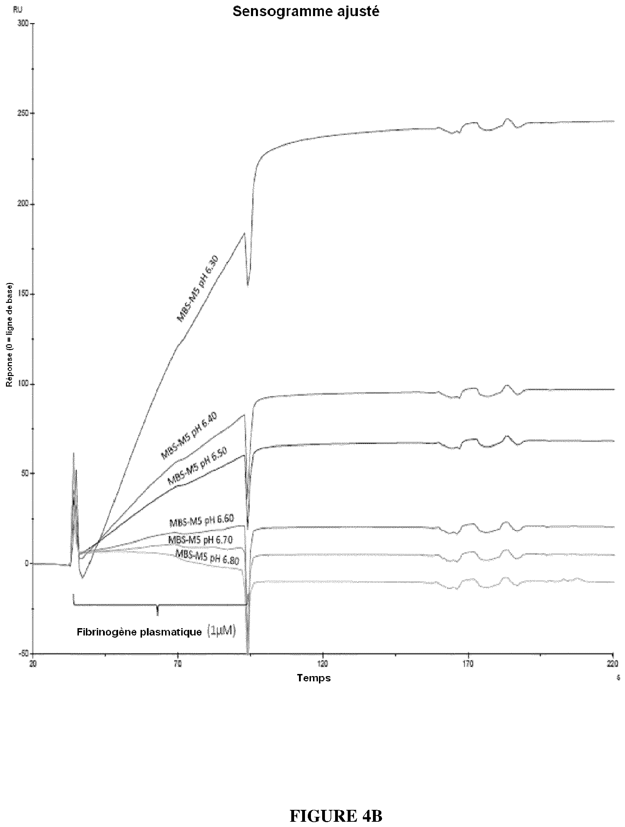

La **figure 4B** représente des sensogrammes SPR illustrant la dépendance vis-à-vis du pH de l'affinité de liaison de l'aptamère de SEQ ID N° : 67 (la séquence de base de SEQ ID N° : 58) au fibrinogène plasmatique humain. Aucune liaison n'est observée pour un pH supérieur à 6,8. Axe des X : temps en s. Axe des Y : réponse SPR en unité arbitraire.

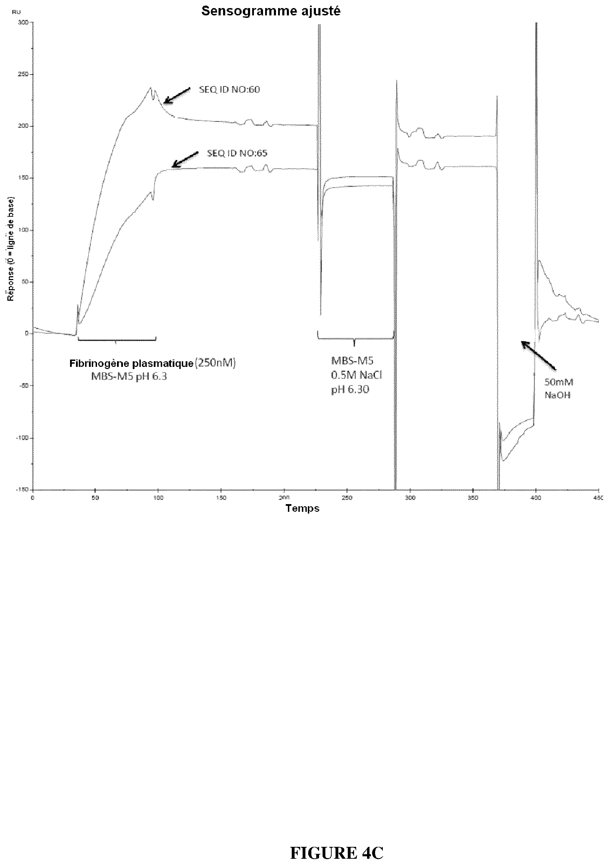

La **figure 4C** représente la courbe de liaison d'un fibrinogène plasmatique humain (sensogramme) pour des aptamères de SEQ ID N° : 60 et SEQ ID N° : 65 (appartenant au second sous-groupe d'aptamères de l'invention) immobilisés sur une puce, obtenus par la technologie SPR. Du fibrinogène plasmatique humain purifié (250 nM) a été injecté à pH 6,3, de sorte qu'un complexe s'est formé comme le montre l'augmentation du signal. L'injection d'une solution tampon à pH 6,3 comprenant du NaCl 0,5 M n'a pas significativement induit l'élution du fibrinogène plasmatique humain. Le support solide a ensuite été régénéré par injection d'une solution de NaOH 50 mM. Axe des X : temps en s. Axe des Y : réponse SPR en unité arbitraire.

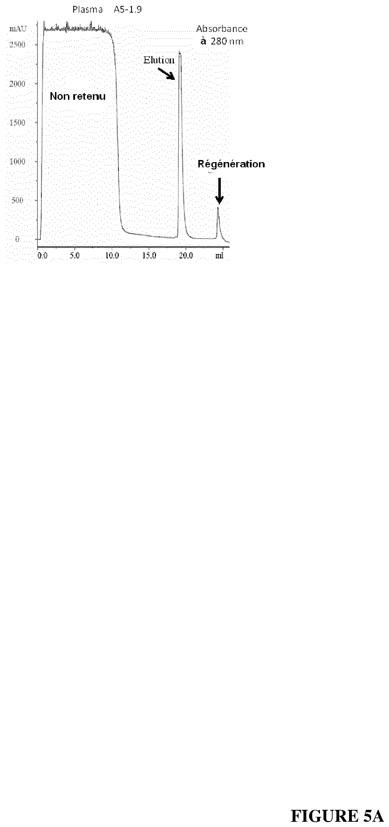

La **figure 5A** représente le profil chromatographique de la purification du fibrinogène provenant du plasma sur un support d'affinité sur lequel est greffé un aptamère de SEQ ID N° : 66. Axe des Y : absorbance à 280 nm. Axe des X : en mL.

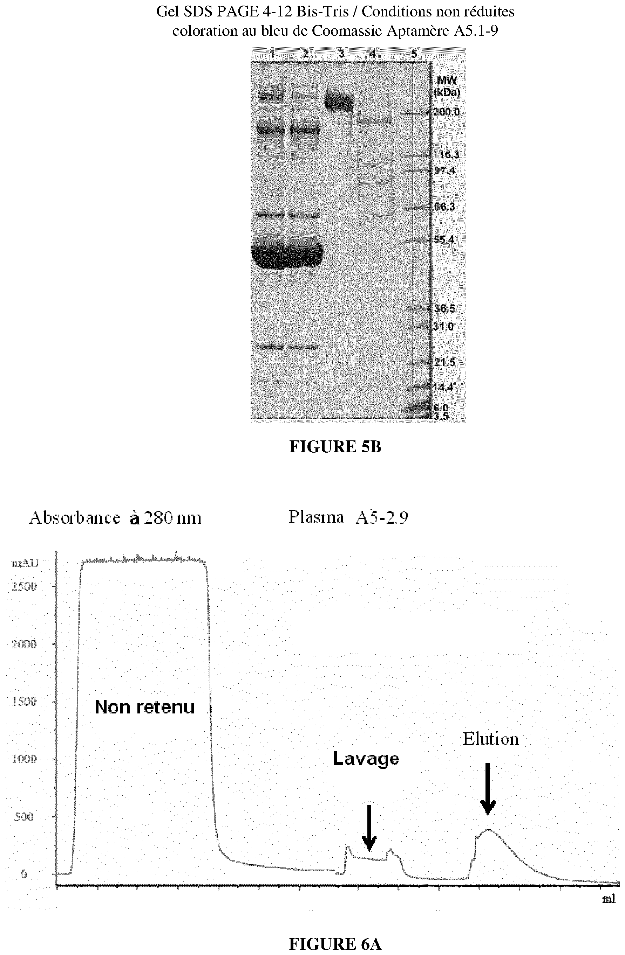

La **figure 5B** représente la photo des gels d'électrophorèse après une coloration au bleu de coomassie dans des conditions non réduites. De la gauche vers la droite ; 1 : le plasma ; 2 : la fraction provenant du plasma qui n'a pas été retenue sur la phase stationnaire, 3 : fraction d'élution contenant du fibrinogène obtenue par chromatographie du plasma, 4 : fraction obtenue après régénération du support stationnaire, et 5 : marqueurs de masse moléculaire. La pureté de la fraction d'élution du fibrinogène était supérieure à 95 % rapportée à la quantité totale de protéines contenue dans la fraction. Le support d'affinité utilisé dans la chromatographie a été greffé avec des aptamères de SEQ ID N° : 66.
La **figure 6A** représente le profil chromatographique de la purification de fibrinogène provenant d'un plasma sur un support d'affinité sur lequel est greffé un aptamère de SEQ ID N° : 67. Axe des Y : absorbance à 280 nm. Axe des X : en mL.

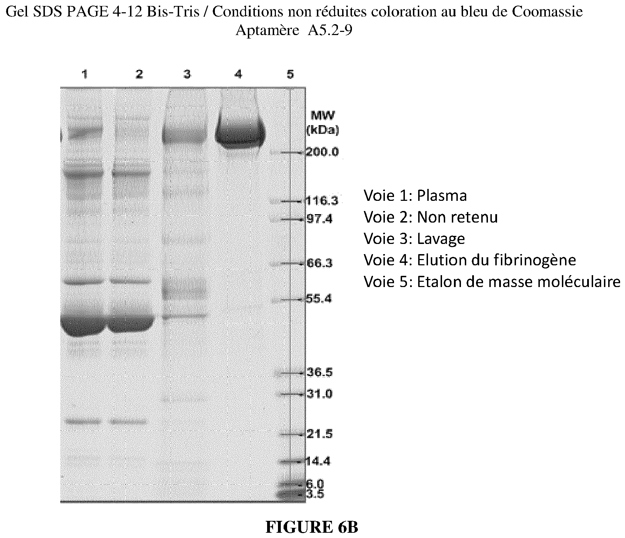

La **figure 6B** représente la photo des gels d'électrophorèse après une coloration au bleu de coomassie dans des conditions non réduites. De la gauche vers la droite ; 1 : le plasma ; 2 : la fraction provenant du plasma qui n'a pas été retenue sur la phase stationnaire, 3 : fraction obtenue après lavage du support stationnaire, 4 : fraction d'élution contenant du fibrinogène obtenue par chromatographie du plasma, et 5 : marqueurs de masse moléculaire. La pureté de la fraction d'élution du fibrinogène était d'au moins 95 % rapportée à la quantité totale de protéines contenue dans la fraction. Le support d'affinité utilisé dans la chromatographie a été greffé avec des aptamères de SEQ ID N° : 67.

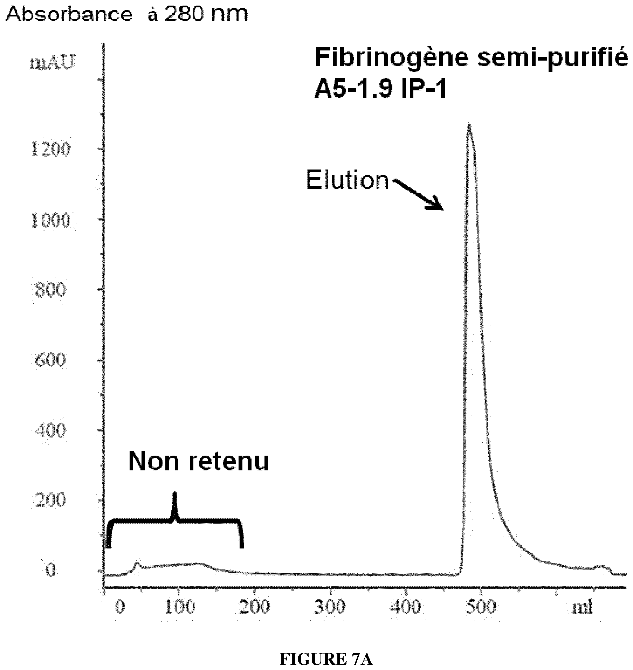

La **figure 7A** représente le profil chromatographique obtenu pour la purification de fibrinogène semi-purifié sur un support d'affinité sur lequel est greffé un aptamère de SEQ ID N° : 66. Axe des Y : absorbance à 280 nm. Axe des X : en mL.

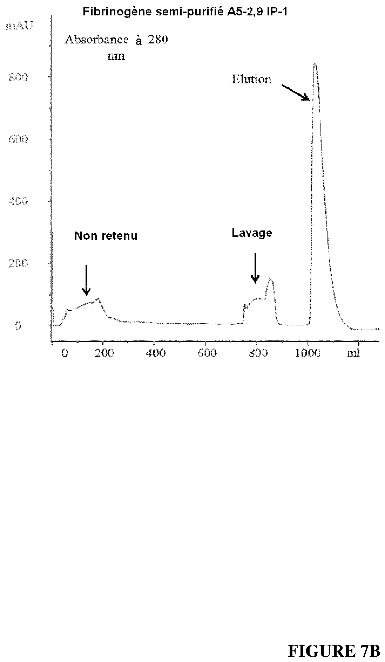

La **figure 7B** représente le profil chromatographique obtenu pour la purification de fibrinogène semi-purifié sur un support d'affinité sur lequel est greffé un aptamère de SEQ ID N° : 67. Axe des Y : absorbance à 280 nm. Axe des X : en mL.

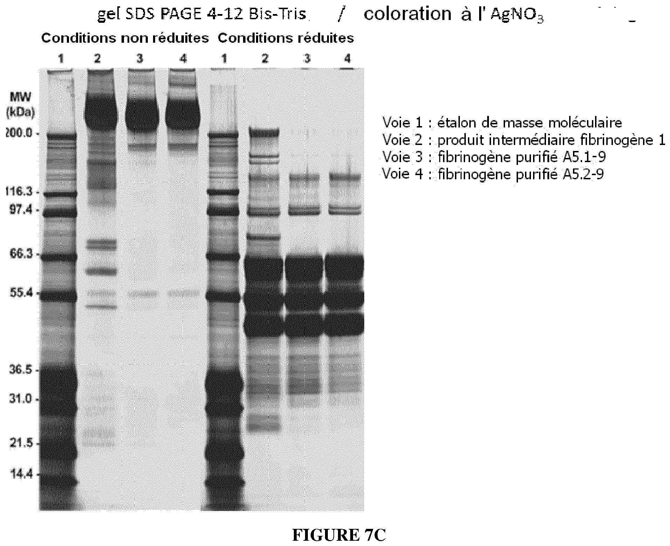

La **figure 7C** représente l'analyse des fractions par SDS-PAGE dans des conditions réduites et non réduites, avec une coloration à l'AgNO₃, des fractions d'élution obtenues par purification de fibrinogène intermédiaire sur les supports d'affinité. Piste 1 : étalon de masse moléculaire, Piste 2 : intermédiaire de fibrinogène (matériau de départ), Piste 3 : Fraction d'élution obtenue avec le support d'affinité N°1 (aptamères de SEQ ID N° : 66), Piste 4 : Fraction d'élution obtenue avec le support d'affinité N°1 (aptamères de SEQ ID N° : 67).

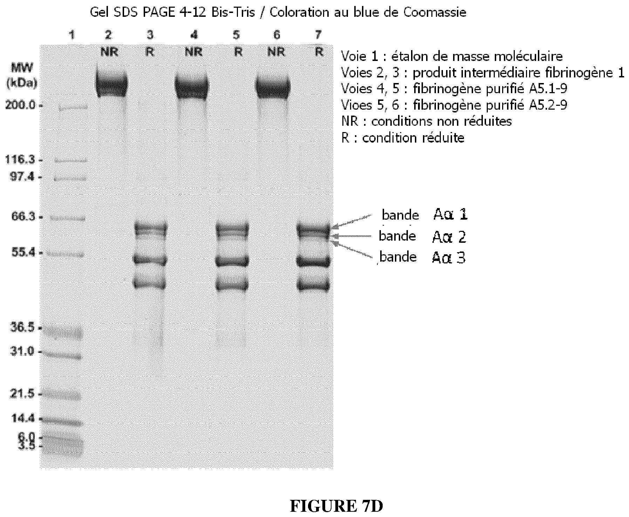

La **figure 7D** représente l'analyse des fractions par SDS-PAGE dans des conditions réduites et non réduites, avec une coloration au coomassie, des fractions d'élution obtenues par purification de fibrinogène intermédiaire sur les supports d'affinité. Piste 1 : étalon de masse moléculaire, Pistes 2 et 3 : intermédiaire de fibrinogène (matériau de départ), Piste 4 et 5 : Fraction d'élution obtenue avec le support d'affinité N°1 (aptamères de SEQ ID N° : 66), Pistes 6 et 7 : Fraction d'élution obtenue avec le support d'affinité N°1 (aptamères de SEQ ID N° : 67). NR : non réduit. R : réduit.

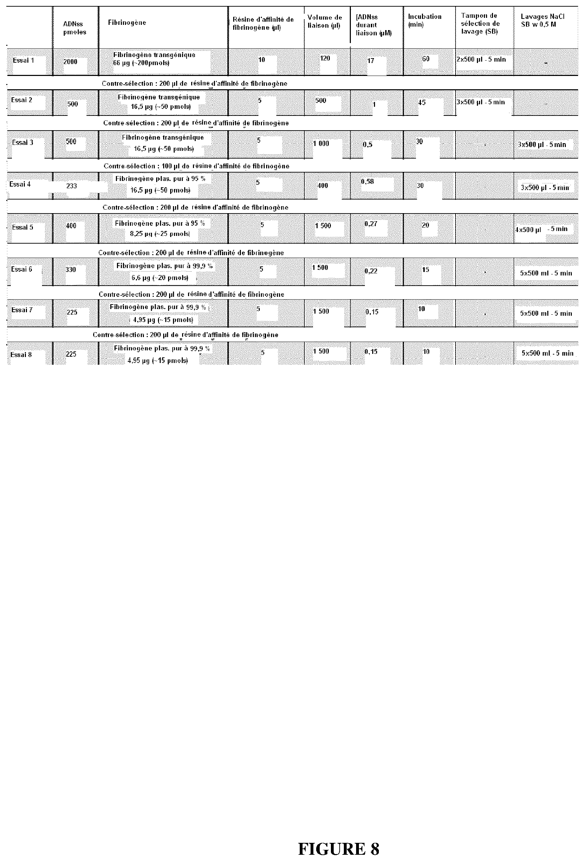

La **figure 8** représente le protocole SELEX utilisé pour identifier des aptamères dirigés contre le fibrinogène humain.

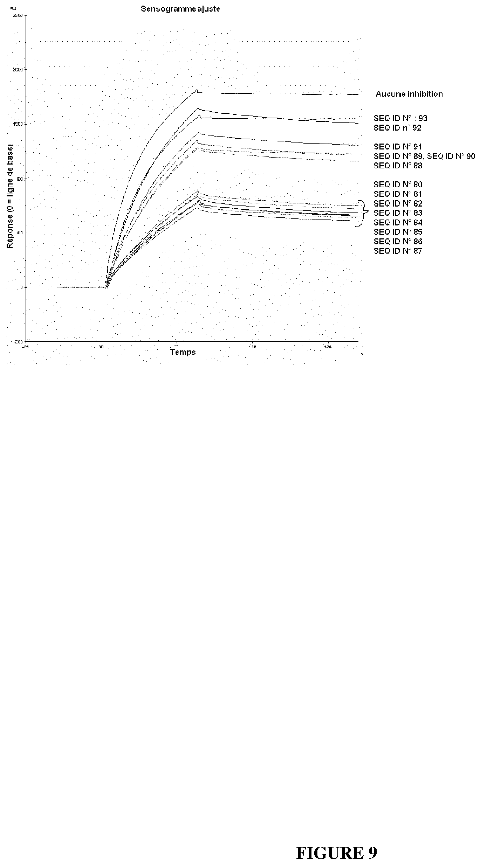

La **figure 9** représente la liaison compétitive d'un aptamère de SEQ ID N° : 66 immobilisé avec un fibrinogène injecté en présence de variants d'aptamères. Ici, plus l'affinité du variant pour le fibrinogène est élevée (par rapport à l'aptamère de SEQ ID N° : 66), plus la réponse pendant l'injection du mélange variant/fibrinogène est faible. Les variants de SEQ ID N° : 66 comprenant l'une des combinaisons de délétion (i) 1/2, (ii) 19/20/21, (iii) 18/19/20/21, (iv) 15/16/19/20 et (v) 14/15/16/20/21/22 suivantes présentent une forte affinité pour le fibrinogène.

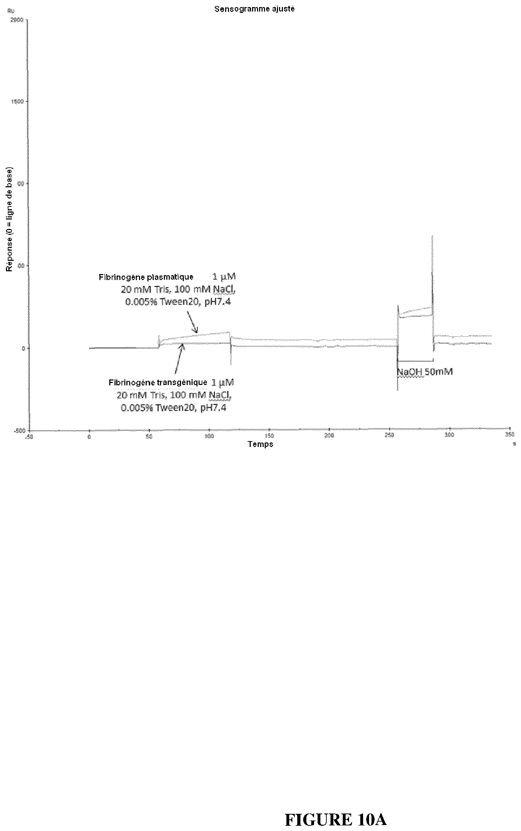

La **figure 10A** représente les courbes de liaison de fibrinogène plasmatique et transgénique humain (sensogramme) pour un aptamère provenant de Base Pair Biotechnologies (référence 6F01 oligo#370) immobilisé sur une puce de capteur, obtenu par la technologie SPR. Le fibrinogène plasmatique et transgénique humain (1000 nM) a été injecté à pH 7,40 à l'aide du tampon recommandé par Base Pair Biotechnologies. Des niveaux de liaison très faibles ont été observés pour le fibrinogène plasmatique et transgénique humain. Le support solide a ensuite été régénéré par injection d'une solution de NaOH 50 mM. Axe des X : temps en s. Axe des Y : réponse en unité arbitraire.

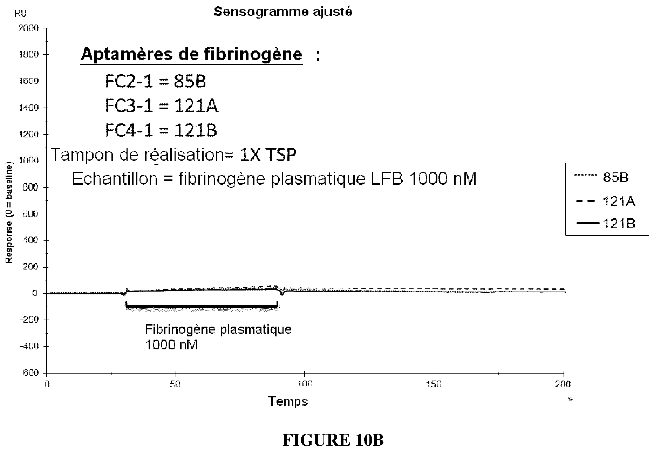

La **figure 10B** représente les courbes de liaison de fibrinogène plasmatique humain (sensogramme) pour 3 aptamères (aptamères 85A, 121A et 121B) décrits dans les données complémentaires de Li et al. (J Am Chem Soc, 2008, 130 (38):12636-12638) immobilisés sur une puce de capteur, obtenus par la technologie SPR. Le fibrinogène plasmatique humain (1000 nM) a été injecté à pH 7,40, comme recommandé par Li et al. Des niveaux de liaison très faibles ont été observés pour le fibrinogène plasmatique et transgénique humain. Le support solide a été ensuite régénéré par injection d'une solution de NaOH 50 mM. Axe des X : temps en s. Axe des Y : réponse en unité arbitraire.

### Remarques :

Le tampon MBS désigne 50 mM de MOPS / 150 mM de NaCl.

Le tampon de NaCl 1M MBS désigne 50 mM de MOPS / 1 M de NaCl.

Le tampon M5 MBS désigne : 50 mM de MOPS pH 6,30 / 150 mM de NaCl / 5 mM de MgCl₂.

Le tampon 0,5M M5 MBS désigne 50 mM de MOPS pH 6,30 / 0,5 M de NaCl / 5 mM de MgCl₂.

### DESCRIPTION DETAILLEE DE L'INVENTION

Le fibrinogène est une protéine constitué d'un dimère de trois chaines polypeptidiques dénommées alpha, beta et gamma. Le fibrinogène est donc un dimère et chaque monomère est composé de trois chaînes (alpha, bêta, gamma). La forme principale de fibrinogène a un poids moléculaire (PM) de 340 kDa. Le fibrinogène est formé de deux sous-unités identiques reliées par des ponts disulfures, donnant à la molécule une forme de fibre comportant 3 globules : un central (domaine E) et deux distaux (domaines D). La molécule entière contient 2964 acides aminés : 610 acides aminés pour la chaîne alpha (α), 461 acides aminés pour la chaîne beta (β), et 411 acides aminés pour la chaîne gamma (γ).Le fibrinogène intervient dans l'hémostase primaire ainsi que dans la coagulation. Il est le plus souvent prescrit pour le traitement des complications associées à l'afibrinogénémie constitutionnelle ou sévère et les syndromes hémorragiques ou risques d'hémorragies associés à une hypofibrinogénémie.

Le terme « stable » correspond à la stabilité physique et/ou chimique de la composition comprenant le fibrinogène. Le terme « stabilité physique » se réfère à la réduction ou l'absence de formation d'agrégats insolubles ou solubles des formes dimériques, oligomériques ou polymériques du fibrinogène, à la réduction ou l'absence de formation de précipité, ainsi qu'à la réduction ou l'absence de toute dénaturation structurale de la molécule. Le terme « stabilité chimique » se réfère à la réduction ou l'absence de toute modification chimique de la composition comprenant le fibrinogène pendant le stockage, à l'état liquide, dans des conditions accélérées.

Un test de stabilité peut être effectué en différentes conditions de température, d'humidité, et de lumière. Préférentiellement, dans le cadre de la présente invention, le test de stabilité peut durer au minimum 1 semaine, préférentiellement au moins 1 mois, préférentiellement au moins 2 mois, préférentiellement au moins 3 mois, préférentiellement au moins 4 mois, préférentiellement au moins 5 mois, plus préférentiellement au moins 6 mois.

Typiquement, la mesure des paramètres de stabilité, tel que définis ci-après, ont lieu
- avant la mise en test de stabilité d'une composition comprenant du fibrinogène ; on peut alors parler de taux initial; et
- pendant ou à l'issue dudit test de stabilité,
étant entendu que ledit test de stabilité peut durer au minimum 1 semaine, préférentiellement au moins 1 mois, préférentiellement au moins 2 mois, préférentiellement au moins 3 mois, préférentiellement au moins 4 mois, préférentiellement au moins 5 mois, plus préférentiellement au moins 6 mois.

La stabilité d'une composition comprenant du fibrinogène peut s'évaluer par inspection visuelle à l'aide notamment d'une mireuse pharmacopée européenne (opalescence, formation de particules), par mesure de la turbidité au moyen d'un spectrophotomètre mesurant l'absorbance ou densité optique à 400 nm, permettant de mesurer les particules en solution de tailles comprises entre 1 nm et 1 µm environ.

Dans un mode de réalisation, la stabilité de la composition comprenant du fibrinogène est définie par la mesure du taux de monomères conservés pendant le test de stabilité au moyen de la méthode High Pressure Size Exclusion Chromatography (HPSEC) ou Dynamic Light Scattering (DLS). Ces méthodes sont bien connues de l'homme du métier.

Une composition de fibrinogène est avantageusement considérée comme stable si la quantité de monomères de fibrinogène conservés pendant la mise en test de stabilité est supérieure à 50%, préférentiellement supérieure à 60%, préférentiellement supérieure à 70%, préférentiellement supérieure à 80%, préférentiellement supérieure à 90%, préférentiellement supérieure à 95% du taux initial de monomères de fibrinogène.

Plus préférentiellement, la quantité de monomères de fibrinogène conservés pendant le test de stabilité est supérieure à 70% du taux initial de monomères de fibrinogène.

Par "taux initial de monomère de fibrinogène", on entend le taux de monomère observé avant la mise en test de stabilité. Typiquement, la quantité de monomère de fibrinogène est mesurée avant la mise en test de stabilité et pendant ou à l'issue dudit test de stabilité.

Alternativement, une composition de fibrinogène est considérée comme stable si la variation de quantité de monomères de fibrinogène durant le test de stabilité est inférieure à 20 %, préférentiellement inférieure à 10%, préférentiellement inférieure à 5%, préférentiellement inférieure à 1%.

Dans un autre mode de réalisation, la stabilité de la composition comprenant du fibrinogène est définie par la mesure du taux de polymères de fibrinogène formés pendant la mise en test de stabilité au moyen de HPSEC. Les polymères de fibrinogènes sont des polymères comprenant au moins 2 chaînes polypeptidiques alpha, 2 chaînes polypeptidiques beta et 2 chaines polypeptidiques gamma du fibrinogène. Ce terme inclue également les trimères de fibrinogène.

Une composition de fibrinogène est avantageusement considérée comme stable si la quantité de polymères de fibrinogène formés pendant la mise en test de stabilité est inférieure à 50%, préférentiellement inférieure à 40%, préférentiellement inférieure à 30%, préférentiellement inférieure à 20%, préférentiellement inférieure à 10% par rapport au taux initial de polymères de fibrinogène. Typiquement, le taux initial de polymères de fibrinogène correspond à l'ensemble des formes polymériques (trimères et plus) du fibrinogène avant la mise en test de stabilité.

Plus préférentiellement, la quantité de polymères de fibrinogène formés pendant la mise en test de stabilité est inférieure à 30%. Typiquement, la quantité de polymères de fibrinogène est mesurée avant la mise en test de stabilité et pendant ou à l'issue dudit test de stabilité.

Alternativement, une composition de fibrinogène est considérée comme stable si la variation de quantité de polymères de fibrinogène durant le test de stabilité est inférieure à 20%, préférentiellement inférieure à 10%, préférentiellement inférieure à 5%, préférentiellement inférieure à 1%.

Dans un autre mode de réalisation, la stabilité de la composition comprenant du fibrinogène est évaluée par la mesure de l'activité coagulable du fibrinogène rapportée à la mesure antigénique du fibrinogène (aussi appelée activité spécifique). De manière particulièrement avantageuse, la composition de fibrinogène stable présente un ratio fibrinogène coagulable / fibrinogène antigène supérieur à 0,5; préférentiellement supérieur à 0,6; supérieur à 0,7; supérieur à 0,8; supérieur à 0,9; encore plus préférentiellement environ égal à 1,0. De manière plus avantageuse encore, une composition de fibrinogène est considérée comme stable si le rapport de son activité coagulable par son activité antigénique à l'issue du test est supérieure à au moins 60%, préférentiellement au moins 70%, 80%, 90%, 95%, 98% ou 99% à la valeur initiale de ce rapport (sa valeur avant test de stabilité).

Par « fibrinogène coagulable » on entend la mesure du fibrinogène fonctionnel par une technique de coagulation, déterminée selon la méthode de von Clauss. L'activité coagulable est exprimée eu g/L de solution de fibrinogène. Cette technique est connue de l'homme du métier qui peut se référer à la publication Von Clauss, A. (1957) Gerinnungsphysiologische schnellmethode zur bestimmung des fibrinogens. Acta Haematologica, 17, 237-246.

Par « fibrinogène antigène » on entend la quantité de fibrinogène, qu'il soit actif ou non, mesurée par méthode néphélémétrique. La quantité de fibrinogène antigénique est exprimée en g/L.

La stabilité de la composition comprenant du fibrinogène est également évaluée par la mesure en SDS PAGE de la conservation des chaînes alpha, beta et gamma de fibrinogène, préférentiellement avant et après un test de stabilité comme défini dans le cadre de la présente invention. Ainsi, une composition de fibrinogène est avantageusement considérée comme stable si :
- l'ensemble des chaînes alpha est conservée à au moins 50%, préférentiellement au moins 60%, préférentiellement au moins 70%, préférentiellement au moins 80%, préférentiellement au moins 90%; plus préférentiellement conservée à environ 100%, et/ou
- l'ensemble des chaînes beta est conservée à au moins 50%, préférentiellement au moins 60%, préférentiellement au moins 70%, préférentiellement au moins 80%, préférentiellement au moins 90%; plus préférentiellement conservée à environ 100%, et/ou
- l'ensemble des chaînes gamma est conservée à au moins 50%, préférentiellement au moins 60%, préférentiellement au moins 70%, préférentiellement au moins 80%, préférentiellement au moins 90%; plus préférentiellement conservée à environ 100%.

En particulier, la composition de fibrinogène est considérée stable si les profils des chaînes alpha et gamma sont conservés à l'issue du test de stabilité. Par conservation des profils des chaînes alpha et gamma on entend la conservation de la répartition des différentes formes des chaînes alpha et gamma.

Ainsi on considère le profil des chaînes alpha conservé au cours du test de stabilité si la répartition des formes Aα1, Aα2 et Aα3 de la chaîne alpha est conservée entre la mesure initiale effectuée avant la mise en stabilité et celle effectuée à l'issue de la période de mise en stabilité. Une composition de fibrinogène peut être considérée comme stable si l'importance de la forme Aα1 (exprimée en pourcentage de l'ensemble des formes des chaînes alphas) après mise en stabilité correspond à au moins 50%, de préférence à au moins 60%, 70%, 80% ou 90% de l'importance de la forme Aα1 (exprimée en pourcentage de l'ensemble des formes des chaînes alphas) avant mise en stabilité.

Ainsi on considère le profil des chaînes gamma conservé au cours du test de stabilité si la répartition des formes γ' et y de la chaîne gamma est conservée entre la mesure initiale effectuée avant la mise en stabilité et celle effectuée à l'issue de la période de mise en stabilité.

La stabilité de la composition comprenant du fibrinogène est également définie par la mesure de la turbidité au moyen de la spectrophotométrie UV à 400nm. En effet, la turbidité reflète la quantité en matière qui trouble la solution. Une composition de fibrinogène est avantageusement considérée comme stable si la turbidité mesurée après le test de stabilité comme défini dans la présente invention est comparable à la turbidité mesurée avant stabilité.

Avantageusement, la turbidité mesurée après la mise en test de stabilité correspond à moins de 130%, moins de 120%, moins de 110% ; avantageusement correspond à 100% de la turbidité mesurée avant stabilité.

Dans un mode de réalisation avantageux de l'invention, la composition comprenant du fibrinogène est stable pendant au moins 1 mois, au moins 2 mois, au moins 3 mois, au moins 4 mois, au moins 5 mois, au moins 6 mois à 4°C.

Par «composition selon l'invention», on entend désigner la composition comprenant du fibrinogène, ladite composition étant stable sous forme liquide. Préférentiellement, ladite composition est constituée de fibrinogène, d'arginine et de citrate.

Par « composition de fibrinogène sous forme liquide » on entend une composition comprenant du fibrinogène en solution, préférentiellement qui n'a pas été soumise à une étape de lyophilisation, dessiccation, déshydratation ou séchage, et qui n'a donc pas besoin d'être reconstituée avant utilisation.

Un objet de l'invention est donc une composition pharmaceutique comprenant du fibrinogène stable sous forme liquide non reconstituée après lyophilisation.

Un autre objet de l'invention est une composition pharmaceutique comprenant du fibrinogène et un ou des excipients pharmaceutiquement acceptables, stable sous forme liquide.

Le terme « excipient pharmaceutiquement acceptable » correspond à tout excipient avantageusement utilisable pour la formulation de protéines humaines, notamment aux substances choisies parmi les sels, les acides aminés, les sucres, les tensioactifs ou tout autre excipient.

Les excipients pharmaceutiquement acceptables de l'invention excluent notamment l'isoleucine, la glycine, et le NaCl.

Avantageusement, les excipients pharmaceutiquement acceptables selon l'invention incluent l'arginine et/ou le citrate. La Demanderesse a mis en évidence qu'il était possible d'obtenir des compositions particulièrement stables dans le temps sous forme liquide comprenant du fibrinogène, de l'arginine et du citrate. Une telle composition est optimale, car elle limite le nombre d'excipients et donc le risque d'effets secondaires dus aux composants de la formulation tout en permettant la conservation sous forme liquide de la composition prête à l'emploi.

Ainsi, dans un mode de réalisation préféré, l'invention concerne une composition comprenant, préférentiellement constitué, du fibrinogène, de l'arginine, et du citrate, et qui est stable sous forme liquide.

Préférentiellement, ledit fibrinogène est un fibrinogène humain.

Selon l'invention, on peut utiliser plusieurs sources de matière première contenant du fibrinogène. La composition de fibrinogène peut ainsi être issue de plasma sanguin autrement dénommées fractions plasmatiques, de surnageant de culture cellulaire ou de lait d'animaux transgéniques.

Dans un mode de réalisation préféré, la composition de l'invention n'a subi aucune étape préalable de lyophilisation, dessiccation, déshydratation ou séchage.

Dans un mode de réalisation préféré, la composition de l'invention n'a subi aucune étape préalable de reconstitution d'un lyophilisat.

Dans un mode de réalisation particulier, la composition selon l'invention est une fraction plasmatique, de préférence une fraction plasmatique obtenue à partir de plasma sanguin prépurifié.

Par « fraction plasmatique obtenue à partir de plasma sanguin prépurifié», on entend toute partie ou sous-partie du plasma sanguin humain, ayant fait l'objet d'une ou plusieurs étapes de purification. Lesdites fractions plasmatiques incluent ainsi le surnageant de plasma cryoprécipité, le cryoprécipité de plasma (remis en suspension), la fraction I obtenue par fractionnement éthanolique (selon la méthode de Cohn ou de Kistler & Nitschmann), les éluats de chromatographies et les fractions non adsorbées des colonnes de chromatographie, y compris des chromatographies multicolonnes, et les filtrats.

Dans un mode de réalisation préféré de l'invention, la composition selon l'invention est issue d'un éluat de chromatographie ou d'une fraction non adsorbée de colonne de chromatographie, y compris de chromatographie multicolonnes.

Dans un mode de réalisation encore plus préféré de l'invention, la composition selon l'invention est issue d'un éluat de chromatographie ou d'une fraction non adsorbée de colonne de chromatographie, à l'exception de chromatographie multicolonnes.

Ainsi, dans un mode de réalisation préféré de l'invention, la composition selon l'invention est issue d'une fraction plasmatique obtenue à partir de cryosurnageant ou de cryoprécipité remis en suspension.

Selon l'invention, le « surnageant de plasma cryoprécipité », ou « cryosurnageant », correspond à la phase liquide obtenue après décongélation de plasma congelé (cryoprécipitation). Notamment, le cryosurnageant peut être obtenu par congélation de plasma sanguin à une température comprise entre -10°C et -40°C, puis décongélation douce à une température comprise entre 0°C et +6°C, préférentiellement entre 0°C et +1°C, suivie d'une centrifugation du plasma décongelé pour séparer le cryoprécipité et le cryosurnageant. Le cryoprécipité est un concentré en fibrinogène, fibronectine, facteur von Willebrand et facteur VIII, tandis que le cryosurnageant contient les facteurs du complément, les facteurs vitamine K dépendants tels que la protéine C, la protéine S, la protéine Z, le facteur II, le facteur VII, le facteur IX et le facteur X, du fibrinogène, les immunoglobulines et l'albumine.

Dans un mode de réalisation avantageux de l'invention, la composition selon l'invention est peut être obtenue selon le procédé décrit par la Demanderesse dans la demande EP1739093 ou dans la demande WO2015/136217.

Dans un autre mode de réalisation particulier de l'invention, la composition selon l'invention provient de lait d'animaux transgéniques, par exemple obtenu selon la méthode décrite dans WO00/17234 ou dans WO00/17239.

Dans un mode de réalisation, la composition selon l'invention provient de plasma qui n'a pas été préalablement appauvri en protéines telles que les immunoglobulines ou l'albumine.

Dans un mode de réalisation, la composition de l'invention est susceptible d'être obtenue par le procédé comprenant les étapes suivantes :
- une étape de purification par chromatographie d'affinité;
- au moins une étape de sécurisation biologique; et
- une étape de formulation sous forme liquide.

Dans un mode de réalisation particulier de l'invention, l'étape de purification par chromatographie d'affinité se fait par chromatographie d'affinité utilisant des ligands d'affinité choisis parmi les anticorps, les fragments d'anticorps, les dérivés d'anticorps ou des ligands chimiques tels que des peptides, des peptides mimétiques, des peptoïdes, des nanofitines ou encore des ligands oligonucléotidiques tels que les aptamères.

Dans un mode de réalisation particulier de l'invention, l'étape de purification par chromatographie d'affinité se fait par chromatographie d'affinité utilisant des ligands d'affinité étant des aptamères.

La demanderesse a réalisé sa propre recherche et a identifié une nouvelle famille d'aptamères, dirigés contre le fibrinogène. Cette nouvelle famille d'aptamères a été identifiée par un procédé SELEX en interne conçu par la demanderesse. Ces aptamères se révèlent se lier spécifiquement à la fois au fibrinogène transgénique et plasmatique humain, indépendamment de l'état de glycosylation de la protéine. Les aptamères identifiés par la demanderesse présentent des propriétés uniques en termes de liaison. En particulier, les aptamères utilisés selon l'invention se lient au fibrinogène d'une façon dépendante du pH. Il est à noter qu'ils présentent une affinité de liaison accrue pour le fibrinogène à un pH légèrement acide tel qu'un pH d'environ 6,3, par rapport à un pH supérieur à 7,0, comme 7,4. Ces propriétés conviennent particulièrement à une utilisation en chromatographie d'affinité parce que la formation du complexe entre la protéine à purifier, à savoir le fibrinogène, et l'aptamère, et la libération ultérieure de la protéine du complexe peuvent être contrôlées par modification du pH du tampon d'élution. En particulier, la libération du fibrinogène du complexe peut être réalisée dans des conditions d'élution modérées, qui ne sont pas susceptibles d'altérer les propriétés de la protéine.

### ▪ Aptamères utilisés selon l'invention

Les aptamères sont utilisés selon l'invention comme ligands d'affinité dans la purification du fibrinogène, par exemple par chromatographie. Les aptamères utilisés selon l'invention sont donc dirigés contre le fibrinogène, à savoir capable de se lier spécifiquement au fibrinogène. Les aptamères utilisés selon l'invention se lient au fibrinogène d'une façon pH-dépendante. De préférence, les aptamères utilisés selon l'invention ne se lient pas au fibrinogène à un pH supérieur à 7,0 et se lient au fibrinogène à un pH acide, par exemple à une valeur de pH choisie parmi 6,0 à 6,6, telle que pH 6,3 ± 0,1.

Comme on l'entend ici, un « aptamère » (aussi appelé aptamère nucléique) désigne un polynucléotide monocaténaire synthétique ayant typiquement une longueur de 20 à 150 nucléotides et capable de se lier avec une grande affinité à une molécule cible. Les aptamères sont caractérisés par une ou des conformations tridimensionnelles qui peuvent jouer un rôle clé au niveau de leurs interactions avec leur molécule cible. Par conséquent, l'aptamère utilisé selon l'invention est capable de former un complexe avec le fibrinogène. Les interactions entre un aptamère et sa molécule cible peuvent englober des interactions électrostatiques, des liaisons hydrogène, et une complémentarité de forme d'empilement aromatique.

«Un aptamère qui se lie spécifiquement à sa molécule cible » signifie que l'aptamère présente une grande affinité pour la molécule cible. La constante de dissociation (Kd) d'un aptamère pour sa molécule cible est typiquement de 10-6 à 10-12 M. Le terme « se lie spécifiquement » est utilisé ici pour indiquer que l'aptamère a la capacité à reconnaitre et à interagir spécifiquement avec sa molécule cible, tout en présentant une réactivité détectable relativement faible avec d'autres molécules qui peuvent être présentes dans l'échantillon. De préférence, l'aptamère se lie spécifiquement à sa molécule cible si son affinité est significativement plus élevée pour la molécule cible, que pour d'autres molécules, y compris des molécules structurellement proches de la molécule cible.

Par exemple, un aptamère peut être capable de se lier spécifiquement à une protéine humaine tout en présentant une affinité plus faible pour un homologue de ladite protéine humaine.

Comme on l'entend ici, « un aptamère qui présente une affinité plus faible pour une molécule donnée que pour sa molécule cible » ou « un aptamère qui est spécifique de sa molécule cible par rapport à une molécule donnée » signifie que la Kd de l'aptamère pour ladite molécule donnée est au moins 5 fois, de préférence au moins 10, 20, 30, 40, 50, 100, 200, 500, ou 1000 fois plus élevée que la Kd dudit aptamère pour la molécule cible.

Les aptamères peuvent être un acide désoxyribonucléique (ADN) ou un acide ribonucléique (ARN). Les aptamères peuvent comprendre un ou plusieurs nucléotides chimiquement modifiés. Les nucléotides chimiquement modifiés englobent, sans s'y limiter, les 2'-amino ou 2'-fluoro-nucléotides, la 2'-ribopurine, le phosphoramidite, un acide nucléique verrouillé (LNA), les nucléotides modifiés par de l'acide boronique, le 5-iodo- ou le 5-bromo-uracil, et la désoxyuridine 5-modifiée telle que la benzyl-dU, l'isobutyl-dU, et la napthyl-dU. Pour la désoxyuridine 5-modifiée, on peut se référer à Rohloff et al., Molecular Therapy-Nucleic acids, 2014, 3, e201 (voir la figure 1 page 4), dont la description est incorporée ici à titre de référence. Dans certains modes de réalisation, l'aptamère utilisé selon l'invention est dépourvu de tout nucléotide modifié par de l'acide boronique, en particulier ceux enseignés dans WO2010/019847. Dans certains autres modes de réalisation, l'aptamère utilisé selon l'invention est dépourvu de toute désoxyuridine 5-modifiée. Dans certains modes de réalisation, l'aptamère peut comprendre un nucléotide modifié au niveau de son extrémité 3' et/ou de son extrémité 5' uniquement (c'est-à-dire que le premier nucléotide et/ou le dernier nucléotide de l'aptamère est/sont le/les seuls nucléotide(s) chimiquement modifié(s)). De préférence, ledit nucléotide modifié peut permettre le greffage de l'aptamère sur un support solide, ou le couplage dudit aptamère à un quelconque groupement d'intérêt (par exemple, utile pour une détection ou une immobilisation).

Lorsque la séquence de l'aptamère est identifiée, l'aptamère peut être préparé par un quelconque procédé de routine connu de l'homme de métier, à savoir par une synthèse d'oligonucléotide chimique, par exemple en phase solide.

Comme on l'entend ici, « un aptamère dirigé vers le fibrinogène », « un aptamère dirigé contre le fibrinogène » ou « un aptamère anti-fibrinogène » désigne un polynucléotide monocaténaire synthétique qui se lie spécifiquement au fibrinogène.

Comme on l'entend ici, le terme « fibrinogène » désigne toute protéine ayant la séquence d'acides aminés du fibrinogène sauvage et des variants de celui-ci, indépendamment de l'état de glycosylation. Le terme « fibrinogène » englobe toute isoforme et variant allélique du fibrinogène, ainsi que toute forme glycosylée, toute forme non glycosylée ou toute forme post-traductionnelle du fibrinogène.

Comme on l'entend ici, un variant du fibrinogène sauvage désigne une protéine ayant une identité de séquence d'au moins 80 %, de préférence une identité de séquence d'au moins 85 %, 90 %, ou 95 %, avec ledit fibrinogène sauvage et qui présente une activité biologique similaire à celle dudit fibrinogène sauvage. Par exemple, l'activité de coagulation du fibrinogène peut être mesurée par le procédé de coagulation de van Clauss. Le variant de fibrinogène peut avoir une activité biologique accrue ou réduite par rapport au fibrinogène sauvage correspondant.

Dans certains modes de réalisation, le fibrinogène désigne une protéine ayant la séquence d'acides aminés du fibrinogène sauvage humain ou un variant de celui-ci. Ledit fibrinogène peut être le fibrinogène plasmatique humain, le fibrinogène humain recombinant ou transgénique. Dans certains modes de réalisation, l'aptamère utilisé selon l'invention est capable de se lier au fibrinogène humain, indépendamment de sa glycosylation. Par exemple, un aptamère de l'invention peut être capable de se lier spécifiquement au fibrinogène plasmatique humain et au fibrinogène humain recombinant, par exemple le fibrinogène recombinant obtenu à partir d'un organisme transgénique multicellulaire ou le fibrinogène recombinant obtenu à partir d'une cellule hôte recombinante.

Les aptamères utilisés selon l'invention peuvent être capables de se lier spécifiquement au fibrinogène à un pH légèrement acide, par exemple à pH 6,3.

De préférence, l'aptamère utilisé selon l'invention présente une constante de dissociation (Kd) pour le fibrinogène plasmatique humain ou pour le fibrinogène transgénique humain d'au plus 10-6 M. Typiquement, la Kd des aptamères utilisés selon l'invention pour le fibrinogène humain peut être de 1.10-12 M à 1.10-6 M à un pH d'environ 6,3. La Kd est de préférence déterminée par un essai de résonance plasmonique de surface (SPR) dans lequel l'aptamère est immobilisé sur la puce de biocapteur et le fibrinogène est envoyé sur les aptamères immobilisés, à un pH d'intérêt, et à diverses concentrations, dans des conditions d'écoulement conduisant aux mesures de kon et de koff et donc de la Kd. On peut se référer au protocole fourni dans l'exemple 3.

Dans certains modes de réalisation, l'aptamère utilisé selon l'invention est spécifique du fibrinogène humain, par rapport au fibrinogène non humain.

Dans certains autres modes de réalisation, l'aptamère utilisé selon l'invention est spécifique du fibrinogène humain par rapport à d'autres protéines présentes dans le plasma, telles que des facteurs de coagulation. De préférence, l'aptamère utilisé selon l'invention se lie spécifiquement au fibrinogène par rapport au facteur FII, FXI ou XIII. Dans des modes de réalisation supplémentaires ou alternatifs, l'aptamère utilisé selon l'invention se lie spécifiquement au fibrinogène par rapport à une fibronectine. Dans des modes de réalisation supplémentaires ou alternatifs, l'aptamère utilisé selon l'invention se lie spécifiquement au fibrinogène par rapport à un plasminogène.

De préférence, l'aptamère utilisé selon l'invention ne se lie pas au fibrinogène à un pH de 7,0 ou plus. L'incapacité de l'aptamère utilisé selon l'invention à se lier au fibrinogène à un pH de 7,0 et plus peut être typiquement déterminée par SPR, tel que décrit dans l'exemple 3. Dans le protocole de l'exemple 3, une absence de liaison est représentée par le fait que le signal SPR reste au niveau de la ligne de base après l'injection du fibrinogène dans un tampon tamponné à un pH d'intérêt.

Dans un certain aspect de l'invention, les aptamères peuvent être caractérisés par la présence d'un groupement spécifique dans leur conformation. Par exemple, les aptamères utilisés selon l'invention peuvent comprendre un groupement tel que représenté sur la figure 1A ou sur la figure 2A. Sans vouloir être lié par une quelconque théorie, la demanderesse pense que la présence de ladite conformation bidimensionnelle peut être impliquée dans les interactions spécifiques avec le fibrinogène.

La présence dudit groupement conformationnel spécifique peut provenir de la présence d'un polynucléotide spécifique (appelé « polynucléotide de base » ou « séquence de base ») au sein de la séquence d'aptamère. Comme on l'entend ici, une « séquence de base » d'un aptamère donné comprend typiquement, ou désigne, la séquence minimale provenant dudit aptamère capable de se lier au fibrinogène.

En étudiant les aptamères identifiés par sa propre recherche, la demanderesse a identifié plusieurs séquences de base d'intérêt, entre autres les polynucléotides de SEQ ID N° : 66 et de SEQ ID N° : 67.

La demanderesse a en outre déterminé les groupements de la séquence consensus dans les SEQ ID N° : 58-65. Comme le montre la figure 2B, les aptamères de SEQ ID N° : 58-65 comprennent deux groupements consensus dans leurs séquences, à savoir GTTGGTAGGG (SEQ ID N° : 77) qui est en amont de GGTGTAT (SEQ ID N° : 78). Ces groupements consensus sont situés dans une région des aptamères qui forme une boucle représentée sur la figure 2A pour l'aptamère de SEQ ID N° : 58. Sans vouloir se lier à une quelconque théorie, la demanderesse pense que ce groupement conformationnel peut jouer un rôle dans la liaison desdits aptamères au fibrinogène.

Dans un certain aspect, l'aptamère utilisé selon l'invention est capable de se lier spécifiquement au fibrinogène et possède l'une des caractéristiques suivantes :
- Ledit aptamère comprend un polynucléotide ayant une identité de séquence d'au moins 70 %, par exemple d'au moins 75 %, 80 %, 85 %, 90 %, ou 95 %, avec la séquence de nucléotides de SEQ ID N° : 66, ou
- Ledit aptamère comprend les groupements de nucléotide GTTGGTAGGG (SEQ ID N° : 77) et GGTGTAT (SEQ ID N° : 78), dans lequel le groupement de SEQ ID N° : 77 est de préférence en amont de SEQ ID N° : 78.

Dans certains modes de réalisation, l'aptamère utilisé selon l'invention est capable de se lier spécifiquement au fibrinogène et possède l'une des caractéristiques suivantes :
- Ledit aptamère comprend un polynucléotide ayant une identité de séquence d'au moins 70 % avec la séquence de nucléotides de SEQ ID N° : 66, ou
- Ledit aptamère comprend le groupement de nucléotide de formule (III) :
   5'-[SEQ ID N° : 79]-[X1]-[SEQ ID N° : 77]-[X2]-[SEQ ID N° : 78]-3' (III) dans lequel :
   - [X2] et [X1] désignent indépendamment un nucléotide ou un oligonucléotide d'une longueur de 2 à 5 nucléotides, de préférence d'une longueur de 2 ou 3 nucléotides,
   - [SEQ ID N° : 77] est un oligonucléotide de SEQ ID N° : 77 (à savoir GTTGGTAGGG),
   - [SEQ ID N° : 78] est un oligonucléotide de SEQ ID N° : 78 (à savoir GGTGTAT) et
   - [SEQ ID N° : 79] est un oligonucléotide de SEQ ID N° : 79 (à savoir TGT).

Dans un mode de réalisation particulier, l'aptamère utilisé selon l'invention comprend un polynucléotide qui :
- a une identité de séquence d'au moins 70 % avec au moins une séquence de nucléotides choisie dans le groupe constitué de SEQ ID N° : 67, SEQ ID N° : 68, SEQ ID N° : 69, SEQ ID N° : 70, SEQ ID N° : 71, SEQ ID N° : 72, SEQ ID N° : 73 SEQ ID N° : 74, et SEQ ID N° : 95 et
- comprend le groupement de nucléotide de formule (III) tel que défini ci-dessus.

Par exemple, l'aptamère utilisé selon l'invention peut comprendre un polynucléotide qui a une identité de séquence d'au moins 70 % avec SEQ ID N° : 67 et qui comprend le groupement de nucléotide de formule (III).

Dans un autre aspect, l'aptamère utilisé selon l'invention est capable de se lier spécifiquement au fibrinogène et comprenant un polynucléotide ayant une identité de séquence d'au moins 70 % avec la SEQ ID N° : 66 ou la SEQ ID N° : 67.

Comme on l'entend ici, une identité de séquence d'au moins 70 % englobe une identité de séquence d'au moins 75 %, 80 %, 85 %, 86 %, 87 %, 88 %, 89 %, 90 %, 91 %, 92 %, 93 %, 94 %, 95 %, 96 %, 97 %, ou 98 %. Le « pourcentage d'identité » entre deux séquences de nucléotides (A) et (B) peut être déterminé par comparaison des deux séquences alignées d'une manière optimale, via une fenêtre de comparaison. Ledit alignement des séquences peut être réalisé par des procédés bien connus, par exemple, à l'aide de l'algorithme d'alignement global de Needleman-Wunsch. Lorsque l'alignement est obtenu, le pourcentage d'identité peut être obtenu en divisant le nombre total de résidus d'acide aminé identiques alignés par le nombre total de résidus contenus dans la séquence la plus longue entre les séquences (A) et (B). L'identité de séquence est typiquement déterminée à l'aide d'un logiciel d'analyse de séquence. Pour comparer deux séquences d'acides nucléiques, on peut utiliser, par exemple, l'outil « Emboss needle » pour un alignement de séquences par paires pour fournir un EMBL-EBI et disponible sur http://www.ebi.ac.uk/Tools/psa/emboss_needle/nucleodite.html à l'aide des réglages de défaut : (i) Matrice : ADN complet, (ii) ouverture d'espace : 10, (iii) niveau d'espace : 0,5, (iv) format de sortie : paire, (v) pénalité d'espace terminal : faux, (vi) ouverture d'espace terminal : 10, (vii) niveau d'espace terminal : 0,5.

L'aptamère utilisé selon l'invention a typiquement une longueur de 20 à 150 nucléotides, de préférence une longueur de 30 à 100 nucléotides, par exemple une longueur de 25 à 90 nucléotides, une longueur de 30 à 80 nucléotides ou une longueur de 30 à 60 nucléotides. Par conséquent, l'aptamère utilisé selon l'invention peut avoir une longueur de 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89 ou 90 nucléotides.

Dans un mode de réalisation particulier, l'aptamère utilisé selon l'invention comprend un polynucléotide qui diffère d'un polynucléotide choisi dans le groupe de SEQ ID N° : 66 et de SEQ ID N° : 67 en raison de 1 à 15 modifications de nucléotide, de préférence en raison de 1 à 10 modifications de nucléotide, comme 1, 2, 3, 4, 5, 6, 7, 8, 9, ou 10 modifications de nucléotides.

Comme on l'entend ici, une « modification de nucléotide » désigne la délétion d'un nucléotide, l'insertion d'un nucléotide ou la substitution d'un nucléotide par un autre nucléotide par rapport à la séquence de référence.

Les aptamères utilisés selon l'invention peuvent aussi comprendre des amorces à leurs extrémités 3' et 5' utiles pour leur amplification par PCR. Dans certains modes de réalisation, ces séquences d'amorce peuvent être incorporées ou partiellement incorporées dans la séquence de base et peuvent donc participer à des interactions de liaison avec le fibrinogène. Dans certains autres modes de réalisation, ces séquences d'amorce sont à l'extérieur de la séquence de base et peuvent ne pas jouer un quelconque rôle dans l'interaction de l'aptamère avec le fibrinogène. Dans certains autres modes de réalisation, l'aptamère est dépourvu de séquences d'amorce.

Dans certains modes de réalisation alternatifs ou supplémentaires, l'aptamère utilisé selon l'invention peut comprendre un polynucléotide d'une longueur de 2 à 40 nucléotides lié à l'extrémité 5' et/ou à l'extrémité 3' de la séquence de base.

Dans un certain aspect, l'aptamère utilisé selon l'invention se lie spécifiquement au fibrinogène et répond à la formule (I)

5'-[NUC1]m-[CENTRAL]-[NUC2]n-3' (I)

dans laquelle
n et m sont des nombres entiers choisis indépendamment parmi 0 et 1,
- [NUC1] est un polynucléotide comprenant de 2 à 40 nucléotides, de préférence de 15 à 25 nucléotides
- [NUC2] est un polynucléotide comprenant de 2 à 40 nucléotides, de préférence de 15 à 25 nucléotides et
- [CENTRAL] est un polynucléotide ayant une identité de séquence d'au moins 70 % avec une séquence de nucléotides choisie dans le groupe constitué de SEQ ID N° : 68, SEQ ID N° : 69 SEQ ID N° : 70, SEQ ID N° : 71, SEQ ID N° : 72, SEQ ID N° : 73, SEQ ID N° : 74, SEQ ID N° : 94 et SEQ ID N° : 95.

Lorsque n=m=0, [NUC1] et [NUC2] sont absents et l'aptamère est constitué de la séquence [CENTRAL]. Lorsque n=0 et m=1, [NUC2] est absent et [NUC1] est présent, l'aptamère répond donc à la formule (Ia)

5'-[NUC1]-[CENTRAL]-3',

Lorsque n=1 et m=0, [NUC1] est absent et [NUC2] est présent, l'aptamère répond donc à la formule (Ib)

5'-[CENTRAL]-[NUC2]-3'.

Dans certains modes de réalisation, [NUC1] comprend, ou est constitué d'un polynucléotide de SEQ ID N° : 75 ou d'un polynucléotide qui diffère de SEQ ID N° : 75 en raison de 1, 2, 3 ou 4 modifications de nucléotide. Dans certains autres modes de réalisation supplémentaires, [NUC2] comprend, ou est constitué d'un polynucléotide de SEQ ID N° : 76 ou d'un polynucléotide qui diffère de SEQ ID N° : 76 en raison de 1, 2, 3 ou 4 modifications de nucléotide.

Dans un autre aspect, l'invention concerne un aptamère dirigé contre le fibrinogène et qui a une identité de séquence d'au moins 70 %, telle qu'au moins 75 %, 80 %, 85 %, 90 %, 95 %, 96 %, 97 % ou 98 % avec une séquence de nucléotides choisie dans le groupe constitué de SEQ ID N° : 1 à SEQ ID N° : 67. Par exemple, l'aptamère utilisé selon l'invention peut avoir une identité de séquence d'au moins 70 % avec une séquence de nucléotides choisie parmi SEQ ID N° : 1, SEQ ID N° : 2, SEQ ID N° : 58, SEQ ID N° : 60, SEQ ID N° : 65, SEQ ID N° : 66 et SEQ ID N° : 67.

La demanderesse a réalisé une analyse poussée des séquences et des conformations possibles des aptamères définis ci-dessus. Cette analyse a conduit à l'identification de deux sous-groupes d'aptamères, chaque sous-groupe étant caractérisé par des propriétés structurelles et fonctionnelles spécifiques.

### - Premier sous-groupe d'aptamères utilisés selon l'invention

Le premier sous-groupe d'aptamères englobe des aptamères dirigés contre le fibrinogène qui comprend une séquence de base présentant une grande identité de séquence avec la séquence de base de SEQ ID N° : 66. Ce premier sous-groupe englobe des aptamères de SEQ ID N° : 1-57 et l'aptamère constitué de la séquence de base de SEQ ID N° : 66.

Par conséquent, l'aptamère utilisé selon l'invention se lie spécifiquement au fibrinogène et comprend un polynucléotide ayant une identité de séquence d'au moins 70 %, 75 %, 80 %, 85 %, 90 %, 92 %, 94 %, 95 %, 96 %, 97 % ou 98 % avec SEQ ID N° : 66. De préférence, ledit aptamère a une longueur de 20 à 110 nucléotides, en particulier une longueur de 25 à 100 nucléotides, telle qu'une longueur de 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 ou 99. En particulier, l'aptamère peut avoir une longueur de 35 à 65 nucléotides.

Dans certains modes de réalisation, l'aptamère comprend un polynucléotide de SEQ ID N° : 66, ou un polynucléotide ayant une séquence de nucléotides qui diffère de SEQ ID N° : 66 en raison de 1 à 16 modifications de nucléotide, telles que 1, 2, 3, 4, 5, 6, 7, 8, 9, 10, 11, 12, 13, 14 ou 15 modifications de nucléotide. Comme mentionné ci-dessus, la ou les modification(s) de nucléotide peuvent être d'un type quelconque. Une modification de nucléotide peut être une délétion d'un nucléotide, l'insertion d'un nucléotide ou la substitution/remplacement d'un nucléotide par un autre nucléotide.

L'alignement de la séquence de base de SEQ ID N° : 66 avec des aptamères de SEQ ID N° : 1-57 a montré que certains nucléotides ne sont pas conservés parmi les aptamères appartenant au premier sous-groupe. Lesdites positions englobent les positions 19, 20, 21, 24, 27, 32, 33, 35, 42, 45, 46, 47, 50, 54, 55 et 57, la numérotation désignant une numérotation de nucléotides dans SEQ ID N° : 66.

D'autres modifications, en particulier une ou plusieurs délétions, peuvent être introduites aux positions 1 et 2 de SEQ ID N° : 66 mais aussi dans la troisième tige de la séquence de base de SEQ ID N° : 66, comme le montre la figure 1A. En particulier, la demanderesse a introduit une à six délétions dans la troisième tige de l'aptamère de SEQ ID N° : 66, en particulier aux positions 14-22, sans aucune perte significative d'affinité au fibrinogène par rapport à la séquence de base parent de SEQ ID N° : 66.

Par conséquent, la ou les modification(s) de nucléotide par rapport à la SEQ ID N° : 66 peuvent être présentes en une ou plusieurs de ces positions de nucléotide. Dans certains modes de réalisation, l'aptamère utilisé selon l'invention comprend un polynucléotide qui diffère de la SEQ ID N° : 66 en raison de 1 à 20, de préférence de 1 à 14, en particulier de 1, 2, 3, 4, 5 ou 6 modifications de nucléotide aux positions de nucléotide choisies parmi 1, 2, 11-25, 32-35, 42, 45-47, 50 et 54-58, de préférence aux positions de nucléotide choisies parmi 1, 2, 14-22, 24, 27, 32, 33, 35, 42, 45, 46, 47, 50, 54, 55 et 57, la numérotation se référant à la numérotation de nucléotide dans SEQ ID N° : 66. De préférence, la ou les modification(s) est/sont un ou des remplacement(s) ou délétion(s) de nucléotide.

Dans certains modes de réalisation particuliers, la ou lesdites modification(s) de nucléotide apparaisse(nt) aux positions de nucléotide choisies parmi 20, 35, 42 et 55. Dans certains modes de réalisation particuliers, l'aptamère utilisé selon l'invention peut comprendre un polynucléotide qui diffère de la SEQ ID N° : 66 en raison d'au plus 4 modifications de nucléotide qui apparaissent de préférence aux positions choisies parmi 20, 35, 42 et 55, la numérotation se référant à la numérotation de nucléotide dans SEQ ID N° : 66.

Par exemple, l'aptamère utilisé selon l'invention peut comprendre un polynucléotide de SEQ ID N° : 66, ou un polynucléotide ayant une séquence de nucléotides qui diffère de la SEQ ID N° : 66 en raison de 1, 2 ou 3 modification(s) de nucléotide, de préférence en raison de 1, 2 ou 3 substitution(s) de nucléotide, la ou lesdites modification(s) de nucléotide étant en une ou des position(s) de nucléotide choisie(s) dans le groupe constitué de 19, 20, 21, 24, 27, 32, 33, 35, 42, 45, 46, 47, 50, 54, 55 et 57, la numérotation se référant à la numérotation de nucléotide dans SEQ ID N° : 66.

Dans certains autres modes de réalisation, l'aptamère utilisé selon l'invention peut comprendre le polynucléotide de SEQ ID N° : 66, ou un polynucléotide ayant une séquence de nucléotides qui diffère de la SEQ ID N° : 66 en raison de 1-14 délétion(s) de nucléotide, de préférence en raison de 1, 2, 3, 4, 5, 6 ou 7 délétion(s) de nucléotide, la ou lesdites délétion(s) de nucléotide étant en une ou des position(s) de nucléotide choisie(s) dans le groupe constitué de 1, 2, 14, 15, 16, 17, 18, 19, 20, 21 et 22, la numérotation se référant à la numérotation de nucléotide dans SEQ ID N° : 66.

Dans certains modes de réalisation supplémentaires, l'aptamère utilisé selon l'invention peut comprendre un polynucléotide de SEQ ID N° : 66, ou un polynucléotide ayant une séquence de nucléotides qui diffère de la SEQ ID N° : 66 en raison de l'une des combinaisons de délétions de nucléotide suivantes :
- délétions de nucléotides aux positions 19, 20, and 21,
- délétions de nucléotides aux positions 18, 19, 20 and 21,
- délétions de nucléotides aux positions 15, 16, 19 and 20,
- délétions de nucléotides aux positions 14, 15, 16, 20 and 21, and
- délétions de nucléotides aux positions 14, 15, 16, 20, 21 and 22.
la numérotation se référant à la numérotation de nucléotide dans SEQ ID N° : 66.

En variante ou de plus, les délétions de nucléotide peuvent être présentes aux positions 1 et 2, la numérotation se référant à la numérotation de nucléotide dans SEQ ID N° : 66.

Comme le montre l'exemple 7, la demanderesse a identifié des variants de SEQ ID N° : 66 capables de se lier au fibrinogène d'une manière concurrente.

Dans certains modes de réalisation supplémentaires ou alternatifs, l'aptamère utilisé selon l'invention est un aptamère qui se lie sélectivement au fibrinogène et qui comprend un polynucléotide choisi dans le groupe constitué de SEQ ID N° : 80, SEQ ID N° : 81, SEQ ID N° : 82, SEQ ID N° : 83, SEQ ID N° : 84, SEQ ID N° : 85, SEQ ID N° : 86, SEQ ID N° : 87, SEQ ID N° : 88, SEQ ID N° : 89, SEQ ID N° : 90, SEQ ID N° : 91, SEQ ID N° : 92, et SEQ ID N° : 93 ou ayant une séquence de nucléotides qui diffère en raison de 1, 2, 3, 4 ou 5 modifications de nucléotide par rapport à une séquence choisie dans le groupe constitué de SEQ ID N° : 80-93.

Des variants préférés de SEQ ID N° : 66 englobent des aptamères de SEQ ID N° : 80-87.

Le premier sous-groupe d'aptamères utilisés selon l'invention englobe aussi des aptamères dirigés contre le fibrinogène et qui comprend ayant une identité de séquence d'au moins 80 %, 85 %, 90 %, 92 %, 94 %, 95 %, 96 %, 97 % ou 98 % avec la SEQ ID N° : 1.

En particulier, l'aptamère peut être de SEQ ID N° : 1 ou il peut avoir une séquence de nucléotides qui diffère de la SEQ ID N° : 1 en raison de 1-20, en particulier de 1, 2, 3, 4, 5, 6, 7, 8, 9 ou 10 modifications de nucléotide. La ou lesdites modification(s) de nucléotide peuvent être de préférence présentes au(x) positions décrites ci-dessus pour la SEQ ID N° : 66.

Comme expliqué dans la section ci-dessous intitulée « Procédé d'obtention d'aptamères utilisés selon l'invention », certains aptamères utilisés selon l'invention ont été identifiés à partir d'un mélange de candidats constitué d'une multitude d'ADN monocaténaires (ADNss), dans lesquels chaque ADNss comprend une séquence centrale aléatoire d'environ 20 à 100 nucléotides flanquée de séquences spécifiques d'environ 15 à 40 nucléotides qui jouent le rôle d'amorces pour une amplification par PCR.

Dans certains modes de réalisation alternatifs ou supplémentaires, l'aptamère utilisé selon l'invention répond à la formule (I) dans laquelle :

5'-[NUC1]m-[CENTRAL]-[NUC2]n-3' (I)

dans laquelle :
- [CENTRAL] est un polynucléotide ayant une identité de séquence d'au moins 70 %, de préférence d'au moins 80 %, par exemple d'au moins 85 %, 90 %, 93 %, 95 %, 96 %, 97 %, 98 %, 99 % ou 100 %, avec la SEQ ID N° : 94,
- n et m sont des nombres entiers choisis indépendamment parmi 0 et 1,
- [NUC1] est un polynucléotide comprenant de 2 à 40 nucléotides, de préférence de 15 à 25 nucléotides, et
- [NUC2] est un polynucléotide comprenant de 2 à 40 nucléotides, de préférence de 15 à 25 nucléotides.

Dans un mode de réalisation préféré, m=1 et [NUC1] comprend, ou est constitué d'un polynucléotide de SEQ ID N° : 75 ou qui diffère de SEQ ID N° : 75 en raison de 1, 2, 3 ou 4 modifications de nucléotide.

Un exemple d'aptamère de formule (I) est l'aptamère de SEQ ID N° : 66.

Par conséquent, l'aptamère utilisé selon l'invention répond à la formule suivante :

5'-[NUC1]-[CENTRAL]-[NUC2]n-3'

dans laquelle n est 0 ou 1.

Dans certains autres modes de réalisation, [NUC2] peut comprendre, ou est constitué d'un polynucléotide de SEQ ID N° : 76 ou d'un polynucléotide qui diffère de SEQ ID N° : 76 en raison de 1, 2, 3 ou 4 modifications de nucléotide.

Dans un aspect spécifique, l'aptamère utilisé selon l'invention peut être un aptamère de formule (I) :

5'-[NUC1]m-[CENTRAL]-[NUC2]n-3' (I)

dans laquelle :
- m est 1,
- n est 0 ou 1,
- [NUC1] est un polynucléotide de SEQ ID N° : 75 ou qui diffère de SEQ ID N° : 75 en raison de 1, 2, 3 ou 4 modifications de nucléotide, de préférence des délétions de nucléotide, et
- [NUC2] est un polynucléotide ayant une longueur de 2 à 40 nucléotides, et
- [CENTRAL] est le polynucléotide de SEQ ID N° : 94, ou a une séquence de nucléotides qui diffère de SEQ ID N° : 94 en raison de 1, 2, 3, 4, 5 ou 6 modifications de nucléotide, de préférence des délétions de nucléotide.

Dans certains modes de réalisation, [NUC2] est un polynucléotide de SEQ ID N° : 76, ou a une séquence qui diffère de SEQ ID N° : 76 en raison de 1, 2, 3 ou 4 modifications de nucléotide, lesdites modifications de nucléotide étant de préférence choisies parmi les substitutions de nucléotide et les délétions de nucléotide.

Dans certains modes de réalisation, les aptamères dudit premier sous-groupe peuvent comprendre un groupement de conformation, comme le montrent sur la figure 1A les nucléotides soulignés. Dans un aspect plus général, les aptamères utilisés selon l'invention peuvent avoir une séquence de nucléotides comprenant des domaines de nucléotide capables de former un groupement de conformation comprenant une boucle centrale comprenant de 15 à 19 nucléotides, de préférence 17 nucléotides portant :
- une première tige ayant une longueur de 4 à 6 nucléotides, de préférence de 5 nucléotides,
- une deuxième tige ayant de 2 à 4 nucléotides, de préférence 3 nucléotides reliée à une boucle comprenant de 13 à 15, de préférence 14, nucléotides, et
- éventuellement une troisième tige ayant de 2 à 8, de préférence 7 nucléotides reliée à une boucle comprenant de 2 à 4, de préférence 3, nucléotides.

Dans certains modes de réalisation préférés, la première tige est adjacente à la deuxième tige et séparée par 2 nucléotides provenant de la troisième tige.

Les aptamères appartenant au premier sous-groupe de l'invention peuvent être capables de se lier au fibrinogène à un pH légèrement acide défini ci-dessus, de préférence à un pH d'environ 6,3. En particulier, les aptamères du premier groupe peuvent se lier au fibrinogène d'une façon dépendante du pH.

Dans certains modes de réalisation, lesdits aptamères présentent une affinité accrue pour le fibrinogène à pH 6,3 par rapport à un pH légèrement basique tel que pH 7,4. Dans certains modes de réalisation, ledit aptamère ne se lie pas au fibrinogène à un pH de 7,0 ou plus. Ledit sous-groupe d'aptamères peut être aussi capable de se lier au fibrinogène en présence de Mg2+. Dans certains modes de réalisation, lesdits aptamères peuvent présenter une affinité de liaison pour le fibrinogène qui dépend du pH et/ou de la présence de Mg2+ dans le milieu. Par exemple, l'affinité de liaison de l'aptamère pour le fibrinogène peut être accrue en présence de Mg2+ en une concentration dans la plage de mM, par exemple de 1 à 10 mM, par rapport au même milieu dépourvu de Mg2+. Comme autre exemple, l'aptamère utilisé selon l'invention se lie spécifiquement au fibrinogène à un pH d'environ 6,3 et ne se lie pas au fibrinogène à un pH supérieur à 7,0, tel que pH 7,4.

Ces propriétés sont par exemple illustrées ici pour l'aptamère de SEQ ID N° : 66 dans la section ci-dessous intitulée « Exemples ».

### - Second sous-groupe d'aptamères utilisés selon l'invention

Le second sous-groupe d'aptamères englobe, sans s'y limiter, les aptamères de SEQ ID N° : 58-65 et la séquence de base de SEQ ID N° : 67.

Les aptamères de SEQ ID N° : 58-65 et de SEQ ID N° : 67 comprennent deux groupements consensus dans leurs séquences, à savoir GTTGGTAGGG (SEQ ID N° : 77) qui est en amont de GGTGTAT (SEQ ID N° : 78), comme le montre l'alignement de séquences de la figure 1C. Sans vouloir se lier à une quelconque théorie, la demanderesse pense que ces groupements consensus sont situés dans une région des aptamères qui forme une boucle représentée sur la figure 1B pour l'aptamère de SEQ ID N° : 58. Ce groupement conformationnel peut jouer un rôle dans la liaison desdits aptamères au fibrinogène . Par conséquent, ce second sous-groupe d'aptamères englobe des aptamères qui se lient spécifiquement au fibrinogène et qui comprend les groupements de nucléotide de SEQ ID N° : 77 et de SEQ ID N° : 78, SEQ ID N° : 77 étant en amont de SEQ ID N° : 78 dans la séquence de base dudit aptamère.

De préférence, ledit aptamère comprend un groupement de nucléotide de formule (III) :

5'-[SEQ ID N° : 79]-[X1]-[SEQ ID N° : 77]-[X2]-[SEQ ID N° : 78]-3' dans laquelle :

- [X2] et [X1] désignent indépendamment un nucléotide ou un oligonucléotide d'une longueur de 2 à 5 nucléotides, de préférence d'une longueur de 2 ou 3 nucléotides,
- [SEQ ID N° : 77] est un oligonucléotide SEQ de ID N° : 77 (à savoir GTTGGTAGGG),
- [SEQ ID N° : 78] est un oligonucléotide de SEQ ID N° : 78 (à savoir GGTGTAT) et
- [SEQ ID N° : 79] est un oligonucléotide de SEQ ID N° : 79 (à savoir TGT).

Par exemple, l'aptamère utilisé selon l'invention peut comprendre un groupement de formule (III) dans laquelle X1 désigne un nucléotide, par exemple G ou T, et X2 est un oligonucléotide d'une longueur de 3 nucléotides. Dans certains modes de réalisation, les aptamères peuvent en outre comprendre un polynucléotide ayant une identité de séquence d'au moins 70 %, 75 %, 80 %, 85 %, 90 % ou 95 %, avec une séquence choisie dans le groupe constitué de SEQ ID N° : 67, SEQ ID N° : 68, SEQ ID N° : 69, SEQ ID N° : 70, SEQ ID N° : 71, SEQ ID N° : 72, SEQ ID N° : 73 SEQ ID N° : 74, et SEQ ID N° : 95.

Par conséquent, le second sous-groupe d'aptamères englobe des aptamères qui se lient spécifiquement au fibrinogène et qui comprend un polynucléotide :
- ayant une identité de séquence d'au moins 70 %, 75 %, 80 %, 85 %, 90 % ou 95 %, avec une séquence de nucléotides choisie dans le groupe constitué de SEQ ID N° : 67, SEQ ID N° : 68, SEQ ID N° : 69, SEQ ID N° : 70, SEQ ID N° : 71, SEQ ID N° : 72, SEQ ID N° : 73 SEQ ID N° : 74, et SEQ ID N° : 95, et
- comprenant un groupement de nucléotide de formule (III) :

   5'-[SEQ ID N° : 79]-[X1]-[SEQ ID N° : 77]-[X2]-[SEQ ID N° : 78]-3'
dans laquelle :
- [X2] et [X1] désignent indépendamment un nucléotide ou un oligonucléotide d'une longueur de 2 à 5 nucléotides, de préférence d'une longueur de 2 ou 3 nucléotides,
- [SEQ ID N° : 77] est un oligonucléotide de SEQ ID N° : 77 (à savoir GTTGGTAGGG),
- [SEQ ID N° : 78] est un oligonucléotide de SEQ ID N° : 78 (à savoir GGTGTAT) et
- [SEQ ID N° : 79] est un oligonucléotide de SEQ ID N° : 79 (à savoir TGT).

L'aptamère utilisé selon l'invention a de préférence une longueur de 20 à 150 nucléotides, en particulier une longueur de 25 à 100 nucléotides, telle qu'une longueur de 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 ou 99.

Dans un mode de réalisation supplémentaire ou alternatif, l'aptamère utilisé selon l'invention se lie au fibrinogène et comprend un polynucléotide ayant une identité de séquence d'au moins 70 %, 75 %, 80 %, 85 %, 90 % ou 95 % avec la séquence de base de SEQ ID N° : 67. Dans certains modes de réalisation, l'aptamère utilisé selon l'invention se lie spécifiquement au fibrinogène et comprend un polynucléotide SEQ ID N° : 67, ou qui diffère de SEQ ID N° : 67 en raison de 1, 2, 3, 4, 5 ou 6 modifications de nucléotides.
- Dans un aspect particulier, l'aptamère utilisé selon l'invention se lie spécifiquement au fibrinogène et répond à la formule (I) 5'-[NUC1]m-[CENTRAL]-[NUC2]n-3' dans laquelle n et m sont des nombres entiers choisis indépendamment parmi 0 et 1,
- [NUC1] est un polynucléotide comprenant de 2 à 40 nucléotides, de préférence de 15 à 25 nucléotides,
- [NUC2] est un polynucléotide comprenant de 2 à 40 nucléotides, de préférence de 15 à 25 nucléotides et
- [CENTRAL] est un polynucléotide ayant une identité de séquence d'au moins 70 %, 75 %, 80 %, 85 %, 90 % ou 95 % avec une séquence de nucléotides choisie dans le groupe constitué de SEQ ID N° : 68, SEQ ID N° : 69 SEQ ID N° : 70, SEQ ID N° : 71, SEQ ID N° : 72, SEQ ID N° : 73, SEQ ID N° : 74, SEQ ID N° : 94 et SEQ ID N° : 95 et qui comprend un groupement de nucléotide de formule (III) défini ci-dessus.

Dans certains modes de réalisation, l'aptamère utilisé selon l'invention est un aptamère de formule (I) comprenant au moins 1, 2, 3 ou toutes les caractéristiques suivantes :
- n = m = 1,
- [NUC1] comprend, ou est constitué d'un polynucléotide de SEQ ID N° : 75 ou d'un polynucléotide qui diffère de SEQ ID N° : 75 en raison de 1, 2, 3 ou 4 modifications de nucléotide,
- [NUC2] comprend, ou est constitué d'un polynucléotide de SEQ ID N° : 76 ou d'un polynucléotide qui idffère de SEQ ID N° : 76 en raison de 1, 2, 3 ou 4 modifications de nucléotide,
- [CENTRAL] est un polynucléotide ayant une identité de séquence d'au moins 80 %, de préférence d'au moins 85 %, avec SEQ ID N° : 95 et qui comprend un groupement de nucléotide de formule (III) définie ci-dessus.

Dans un autre aspect alternatif ou particulier, l'aptamère utilisé selon l'invention se lie spécifiquement au fibrinogène et comprend un polynucléotide ayant une identité de séquence d'au moins 70 %, 75 %, 80 %, 85 %, 90 % ou 95 % avec un polynucléotide choisi parmi SEQ ID N° : 58, SEQ ID N° : 59, SEQ ID N° : 60, SEQ ID N° : 61, SEQ ID N° : 62, SEQ ID N° : 63, SEQ ID N° : 64, SEQ ID N° : 65, et SEQ ID N° : 67.

Dans certains modes de réalisation, les aptamères dudit second sous-groupe peuvent comprendre un groupement de conformation, comme le montrent sur la figure 1B les nucléotides soulignés. Dans un aspect plus général, les aptamères utilisés selon l'invention peuvent avoir une séquence de nucléotides comprenant des domaines de nucléotide capables de former un groupement de conformation comprenant une tige ayant une longueur de 2 à 5 nucléotides, par exemple 4 nucléotides, liée à une boucle de 23 à 27 nucléotides. De préférence, la boucle est dépourvue de tout groupement de tige-boucle ou de groupement de tige supplémentaire.

Les aptamères appartenant audit second sous-groupe peuvent être capables de se lier au fibrinogène à un pH légèrement acide, de préférence à un pH d'environ 6,3. Dans certains modes de réalisation, lesdits aptamères présentent une liaison accrue au fibrinogène à pH 6,3 par rapport à un pH supérieur à 7,0, tel que pH 7,4. De préférence, lesdits aptamères ne se lient pas au fibrinogène à un pH supérieur à 7,0. Plus généralement, les aptamères du second sous-groupe peuvent se lier au fibrinogène d'une façon dépendante du pH.

Ce sous-groupe d'aptamères peut être aussi capable de se lier au fibrinogène en l'absence de Mg2+. Dans certains modes de réalisation, lesdits aptamères peuvent présenter une affinité de liaison pour le fibrinogène qui dépend du pH et/ou de la présence de Mg2+ dans le milieu. Par exemple, l'affinité de liaison de l'aptamère pour le fibrinogène peut être réduite en présence de Mg2+, par exemple dans un milieu comprenant du Mg2+ dans la plage de mM, par rapport au même milieu dépourvu de Mg2+.

### - Utilisation des aptamères selon l'invention comme ligands d'affinité

Des ligands d'affinité comprenant un aptamère dirigé contre le fibrinogène peuvent être utilisés selon l'invention. Lesdits ligands d'affinité peuvent être immobilisés sur un support solide pour la purification du fibrinogène.

Typiquement, le ligand d'affinité utilisé selon l'invention comprend (i) un groupement d'aptamère, à savoir un aptamère dirigé contre le fibrinogène tel que défini ci-dessus lié à au moins une (ii) entité non aptamère utile pour l'immobilisation sur un substrat approprié. De préférence, l'entité non aptamère est de préférence liée à l'extrémité 5'- ou 3' de l'aptamère. Dans un certain mode de réalisation, le ligand d'affinité peut comprendre un moyen d'immobilisation lié au groupement d'aptamère directement ou par un groupement spacer . Par conséquent, le ligand d'affinité peut comprendre, ou être constitué d'un composé de formule (IV) :

[IMM]-([SPACER ])p-[APTAMÈRE] dans laquelle

- [APTAMÈRE] désigne un aptamère tel que défini ci-dessus,
- [SPACER] est un groupement spacer,
- [IMM] est un groupement pour l'immobilisation de l'aptamère sur un support, et
- p est 0 ou 1.

p égal 0 signifie que le spacer est absent et que [IMM] est directement lié à [APTAMÈRE], de préférence à l'extrémité 3' ou 5' de l'aptamère.

p égal 1 signifie que le spacer est présent et relie [IMM] et [APTAMÈRE].

Le groupement spacer est typiquement choisi de façon à réduire l'encombrement stérique du groupement d'aptamère et pour améliorer son accessibilité tout en conservant la capacité de l'aptamère à se lier spécifiquement au fibrinogène . Le groupement spacer peut être d'un type quelconque. Le spacer peut être un nucléotide monocaténaire non spécifique, à savoir qui n'est pas capable de se lier à une protéine, y compris le fibrinogène . Typiquement, le spacer peut avoir une longueur de 2 à 20 nucléotides. Des exemples de spacer nucléiques appropriés sont polyA et polyT. Dans certains autres modes de réalisation, le spacer peut être une entité chimique non nucléique. Par exemple, le spacer peut être choisi dans le groupe constitué d'un peptide, d'un polypeptide, d'un oligo- ou d'un polysaccharide, d'une chaîne hydrocarbonée éventuellement interrompue par un ou plusieurs hétéroatomes et éventuellement substituée par un ou plusieurs substituants tels qu'un hydroxyle, des halogènes ou alkyle en C1-C3 ; des polymères y compris des homopolymères, des copolymères et des polymères séquencés, et des combinaisons de ceux-ci. Par exemple, le spacer peut être choisi dans le groupe constitué de polyéthers tels que le polyéthylène glycol (PEG) ou le polypropylène glycol, le poly(alcool vinylique), le polyacrylate, le polyméthacrylate, la polysilicone, et une combinaison de ceux-ci. Par exemple, le spacer peut comprendre plusieurs chaînes hydrocarbonées, oligomères ou polymères liés par un quelconque groupe approprié, tel qu'un hétéroatome, de préférence -O- ou -S-, -NHC(O)-, -OC(O)-, -NH-, -NH-CO-NH-, -O-CO-NH-, un phosphodiester ou un phosphorothioate. Ces chaînes de spacer peuvent comprendre de 2 à 200 atomes de carbone, comme de 5 à 50 atomes de carbone. De préférence, le spacer est choisi parmi les oligonucléotides non spécifiques, les chaînes hydrocarbonées, les polyéthers, en particulier le polyéthylène glycol et des combinaisons de ceux-ci.

Par exemple, le spacer comprend au moins un groupement de polyéthylène glycol comprenant de 2 à 20 monomères. Par exemple, le spacer peut comprendre de 1 à 4 blocs triéthylène glycol liés ensemble par des segments de liaison appropriés. Par exemple, le spacer peut être un dérivé de triéthylène glycol éthylamine hydrophile en C12. En variante, le spacer peut être une chaîne hydrocarbonée en C2-C20, en particulier une chaîne alkyle en C2-C20 telle qu'une chaîne méthylène en C12.

Le spacer est de préférence lié à l'extrémité 3' ou à l'extrémité 5 du groupement d'aptamère. [IMM] désigne un quelconque groupement convenable permettant d'immobiliser le ligand d'affinité sur un substrat, de préférence un support solide. [IMM] dépend du type d'interactions recherché pour immobiliser le ligand d'affinité sur le substrat.

Par exemple, le ligand d'affinité peut être immobilisé grâce à des interactions non covalentes spécifiques y compris des liaisons hydrogène, des forces électrostatiques ou des forces de Van der Waals. Par exemple, l'immobilisation du ligand d'affinité sur le support peut dépendre de couples ligand/anti-ligand (par exemple, anticorps/antigène tel que biotine/anticorps anti-biotine et digoxygénine/anticorps anti-digoxygénine, ou ligand/récepteur) et d'étiquettes de liaison de protéine. Une multitude d'étiquettes de protéine sont bien connues de l'homme de métier et englobe, par exemple, la biotine (pour une liaison à des dérives de streptavidine ou d'avidine), la glutathione (pour une liaison à des protéines ou à d'autres substances liées à la glutathione-S-transférase), le maltose (pour une liaison à des protéines ou à d'autres substances liées à une protéine de liaison à du maltose), les lectines (pour une liaison à des groupements de sucre), une étiquette c-myc, une étiquette d'antigène d'hémaglutinine (HA), une étiquette de thiorédoxine, une étiquette FLAG, une étiquette polyArg, une étiquette polyHis, une étiquette Strep, un domaine de liaison à la chitine, un domaine de liaison à la cellulose, et analogues. Dans certains modes de réalisation, [IMM] désigne la biotine. Par conséquent, le ligand d'affinité utilisé selon l'invention convient à une immobilisation sur des supports sur lesquels est greffée de l'avidine ou de la streptavidine.

En variante, le ligand d'affinité peut convenir à un greffage covalent sur un support solide. [IMM] peut donc désigner une entité chimique comprenant un groupe chimique réactif. L'entité chimique a typiquement une masse moléculaire inférieure à 1000 g.mol-1, de préférence inférieure à 800 g.mol-1, telle qu'inférieure à 700, 600, 500 ou 400 g.mol-1. Les groupes réactifs peuvent être d'un type quelconque et englober une amine primaire, un groupe maléimide, un groupe sulfhydryle, et analogues.

Par exemple, l'entité chimique peut dériver d'un composé SIAB, d'un composé SMCC ou de dérivés de ceux-ci. L'utilisation de sulfo-SIAB pour immobiliser des oligonucléotides est décrite, par exemple, dans Allerson et al., RNA, 2003, 9:364-374.

Dans certains modes de réalisation, [IMM] comprend un groupe amino primaire. Par exemple, [IMM] peut être -NH2 ou aminoalkyle en C1-C30, de préférence aminoalkyle en C1-C6. Un ligand d'affinité dans lequel [IMM] comprend un groupe primaire convenable convient pour une immobilisation sur un support portant des groupes acide carboxyliques activés. Les groupes acide carboxylique activés englobent, sans s'y limiter, un chlorure d'acide, des groupes anhydride et ester mixtes. Un groupe acide carboxylique activé préféré est le N-hydroxysuccinimide ester.

Comme mentionné ci-dessus, [IMM]-([SPACER])p est de préférence lié à l'extrémité 3' ou à l'extrémité 5' de l'aptamère. L'extrémité du groupement d'aptamère qui n'est pas lié à [IMM]-([SPACER])p peut comprendre un nucléotide chimiquement modifié tel que la 2'-o-méthyl- ou 2'-fluoropyrimidine, la 2'-ribopurine, le phosphoramidite, un nucléotide inversé ou un groupe chimique tel que PEG ou le cholestérol. Ces modifications peuvent prévenir la dégradation, en particulier la dégradation enzymatique des ligands. Dans d'autres modes de réalisation, ladite extrémité libre de l'aptamère (à savoir qui n'est pas liée à [IMM] ou à [SPACER]) peut être liée à un moyen de détection tel que décrit ci-dessus.

Un support d'affinité capable de se lier sélectivement au fibrinogène peut aussi être utilisé selon l'invention, il comprend sur ce support une pluralité de ligands d'affinité tels que décrits ci-dessus.

Les ligands d'affinité peuvent être immobilisés sur le support solide par des interactions non covalentes ou par une ou des liaison(s) covalente(s).

Dans certains modes de réalisation, les ligands d'affinité sont greffés par covalence sur ledit support. Typiquement, le greffage est réalisé par réaction du groupe chimique réactif présent dans le groupement [IMM] du ligand avec un groupe chimique réactif présent à la surface du support solide.

De préférence, le groupe chimique réactif du ligand est un groupe amine primaire et celui présent sur le support solide est un groupe acide carboxylique activé tel qu'un groupe carboxylique activé par NHS (à savoir le N-hydroxysuccimidyle ester). Dans ce cas, la réaction de greffage peut être réalisée à un pH inférieur à 6, par exemple à un pH de 3,5 à 4,5, comme illustré dans l'exemple 4 et décrit dans WO2012090183, dont la description est incorporée ici à titre de référence.

Le support solide du support d'affinité peut être d'un type quelconque et est choisi en fonction de l'utilisation envisagée.

Par exemple, le support solide peut être choisi parmi un support en plastique, en métal et inorganique tel que le verre, le nickel/oxyde de nickel, le titane, l'oxyde de zirconium, le silicium, le silicium contraint, le silicium polycristallin, le dioxyde de silicium ou une céramique. Ledit support peut être contenu dans un dispositif tel qu'un dispositif microélectronique, un dispositif microfluide, un capteur, un biocapteur ou une puce, par exemple, convenant à une utilisation en SPR. En variante, le support peut être sous la forme de billes, telles que des billes polymères, métalliques ou magnétiques. Ces supports peuvent convenir des besoins de détection et de diagnostic.

Dans d'autres modes de réalisation, le support solide peut être un gel polymère, un filtre ou une membrane. En particulier, le support solide peut être constitué de gélose, de gélose réticulée, de cellulose ou de polymères synthétiques tels que le polyacrylamide, le polyéthylène, le polyamide, la polysulfone, et les dérivés de ceux-ci. Ces supports peuvent convenir à la purification du fibrinogène. Par exemple, le support solide peut être un support pour une chromatographie, en particulier pour une chromatographie liquide d'affinité. Par exemple, le support d'affinité utilisé selon l'invention peut être approprié à la réalisation d'une chromatographie d'affinité à l'échelle industrielle. Le support d'affinité utilisé selon l'invention peut donc être utilisé comme phase stationnaire dans un procédé de chromatographie, par exemple dans un procédé de chromatographie sur colonne ou dans un procédé de chromatographie discontinu.

### Utilisations des aptamères et des ligands d'affinité selon l'invention dans la purification du fibrinogène

Un procédé de capture du fibrinogène utilisé selon l'invention comprend les étapes suivantes :
- la fourniture d'un support solide sur lequel est immobilisé un aptamère ou un ligand d'affinité utilisé selon l'invention,
- la mise en contact dudit support solide avec une solution contenant le fibrinogène, afin que le fibrinogène soit capturé par la formation d'un complexe entre le fibrinogène et ledit aptamère ou ledit ligand d'affinité immobilisé sur le support solide.

Dans certains modes de réalisation, le procédé peut comprendre une ou plusieurs étapes supplémentaires telles que :
- une étape de libération du fibrinogène dudit complexe,
- une étape de récupération du fibrinogène dudit complexe,
- une étape de détection de la formation du complexe entre le fibrinogène et ledit aptamère ou ligand d'affinité,
- une étape de quantification du fibrinogène.

La détection du complexe et la quantification du fibrinogène (ou celle du complexe) peuvent être réalisées par un quelconque procédé connu de l'homme de métier. Par exemple, la détection et la quantification peuvent être réalisées par SPR, comme illustré dans les exemples.

En variante, on peut utiliser un essai de type ELISA dans lequel un anticorps anti-fibrinogène marqué est utilisé pour la détection ou la quantification du complexe formé entre le fibrinogène et les ligands d'affinité selon l'invention. L'anticorps anti-fibrinogène peut être marqué avec un fluorophore ou couplé à une enzyme convenant à la détection, telle que la peroxydase du raifort.

Comme illustré en détail dans les exemples 5 et 6 ci-dessous, les aptamères utilisés selon l'invention conviennent particulièrement à une utilisation dans la purification du fibrinogène. Un procédé de purification du fibrinogène utilisé selon l'invention à partir d'une composition de départ comprend les étapes suivantes :
a. la mise en contact de ladite composition de départ avec un support d'affinité tel que défini ci-dessus, dans des conditions convenant à la formation d'un complexe entre (i) les aptamères ou les ligands d'affinité immobilisés sur ledit support et (ii) le fibrinogène ,
b. la libération du fibrinogène dudit complexe, et
c. la récupération du fibrinogène sous une forme purifiée.

Un procédé de préparation utilisé selon l'invention d'une composition de fibrinogène purifiée à partir d'une composition de départ contenant le fibrinogène comprend les étapes suivantes:
a. la mise en contact de ladite composition de départ avec un support d'affinité tel que défini ci-dessus, dans des conditions convenant à la formation d'un complexe entre (i) les aptamères ou les ligands d'affinité immobilisés sur ledit support et (ii) le fibrinogène,
b. la libération du fibrinogène dudit complexe, et
c. la récupération de la composition de fibrinogène purifiée.

Comme on l'entend ici, la composition de départ peut être toute composition qui comprend potentiellement le fibrinogène. La composition de départ peut comprendre des contaminants desquels le fibrinogène doit être séparé.

Les contaminants peuvent être d'un type quelconque et dépendent de la nature de la composition de départ. Les contaminants englobent des protéines, des sels, des hormones, des vitamines, des nutriments, des lipides, des débris cellulaires tels que des fragments de membranes cellulaires, et analogues. Dans certains modes de réalisation, les contaminants peuvent comprendre des protéines sanguines telles que des facteurs de coagulation, de la fibronectine, de l'albumine, de l'immunoglobuline, un plasminogène, l'alpha-2-macroglobuline, et analogues.

Dans certains autres modes de réalisation, le contaminant peut comprendre des protéines non humaines, en particulier des protéines non humaines exprimées de façon endogène par un hôte recombinant tel qu'une cellule recombinante, une bactérie ou une levure, ou un animal transgénique.

Typiquement, la composition de départ peut être, ou peut dériver, d'une culture cellulaire, d'un bouillon de fermentation, d'un lysat cellulaire, d'un tissu, d'un organe ou d'un fluide corporel. Comme on l'entend ici, une « composition de départ » dérive d'une entité d'intérêt, telle que le lait, le sang ou une culture cellulaire, ce qui signifie que la composition de départ est obtenue à partir de ladite entité par soumission de ladite entité à une ou plusieurs étapes de traitement. Par exemple, l'entité d'intérêt peut être soumise à un ou plusieurs traitement tels qu'une lyse cellulaire, une étape de précipitation telle que la précipitation d'un sel, une cryo-précipitation ou une floculation, une étape de filtration telle qu'une filtration poussée ou une ultrafiltration, une centrifugation, une clarification, une chromatographie, une étape d'extraction telle qu'une extraction liquide-liquide ou solide-liquide, une inactivation virale, une pasteurisation, des étapes de congélation/décongélation, et analogues. Par exemple, une composition de départ dérivée du sang englobe, sans s'y limiter, le plasma, une fraction de plasma et un cryo-précipité de sang.

Dans certains modes de réalisation, la solution de départ est dérivée de sang, de préférence de sang humain. La composition de départ peut être choisie parmi le plasma, une fraction de plasma, par exemple la fraction I obtenue par un procédé de fractionnement à l'éthanol de Cohn, et un cryo-précipité de sang. Dans certains modes de réalisation, la composition de départ est une fraction de plasma appauvrie en immunoglobuline et/ou une fraction de plasma appauvrie en albumine et/ou une fraction sanguine ou de plasma appauvrie en protéine à coagulation dépendant de la vitamine K.

Dans certains autres modes de réalisation, la composition de départ est obtenue à partir d'un hôte recombinant. De préférence, l'hôte recombinant est un animal transgénique, tel qu'un mammifère transgénique non humain. Le mammifère transgénique non humain peut être un animal quelconque qui a été génétiquement modifié de façon à exprimer le fibrinogène humain. De préférence, le fibrinogène humain est exprimé dans un fluide corporel dudit animal transgénique.

La solution de départ peut donc être, ou peut dériver, d'un fluide corporel d'un animal transgénique. Les fluides corporels englobent, sans s'y limiter, le sang, le lait de sein et l'urine. Dans un mode de réalisation particulier, la composition de départ est, ou dérive, du lait provenant d'un mammifère transgénique non humain. Les procédés de production d'un animal transgénique capable de sécréter une protéine d'intérêt dans le lait sont bien connus dans la technique. Typiquement, ces procédés englobent l'introduction d'un produit d'assemblage génétique comprenant un gène codant pour la protéine d'intérêt liée de façon opérationnelle à un promoteur à partir d'une protéine qui est naturellement sécrétée dans le lait (tel qu'un promoteur de caséine ou un promoteur WHAP) dans un embryon d'un mammifère non humain. L'embryon est ensuite transféré dans l'utérus d'une femelle appartenant à la même espèce animale et qui a été préparée sur le plan hormonal pour une grossesse.

Dans certains modes de réalisation préférés, la composition de départ peut être choisie parmi le sang humain, le lait transgénique et des dérivés de ceux-ci.

Le support d'affinité utilisé dans les procédés de l'invention peut être un support d'affinité quelconque décrit ci-dessus. De préférence, le support d'affinité est un support d'affinité pour la réalisation d'une chromatographie d'affinité. En fait, les procédés de purification du fibrinogène ou de préparation d'une composition purifiée de fibrinogène sont de préférence basés sur des technologies de chromatographie, par exemple des modes discontinus ou sur colonne, dans lesquels le support d'affinité joue le rôle de phase stationnaire. Dans une étape a), un volume approprié de la composition de départ contenant le fibrinogène est mis en contact avec un support d'affinité dans des conditions convenant pour favoriser les interactions spécifiques des groupements d'aptamère anti-fibrinogènes présents à la surface du support d'affinité avec le fibrinogène, de sorte qu'un complexe se forme entre les molécules de fibrinogène et lesdits groupements d'aptamère. Dans l'étape a), le fibrinogène est donc retenu sur le support d'affinité. La liaison entre les groupements d'aptamère et les molécules de fibrinogène peut être renforcée par réalisation de l'étape a) à un pH légèrement acide. Dans certains modes de réalisation, l'étape a) est réalisée à un pH inférieur à 7,0, de préférence inférieure à 6,9, 6,8 ou 6,7. En particulier, l'étape a) peut être réalisée à un pH de 6,0 à 6,8, de préférence à un pH de 6,0 à 6,5, comme 6,0, 6,1, 6,2, 6,3, 6,4 ou 6,5. Par exemple, l'étape a) peut être réalisée à un pH de 6,1 à 6,5, comme un pH de 6,3. Dans un aspect plus général, la condition de pH de l'étape a) peut être choisie de façon à favoriser la liaison du fibrinogène sur le support d'affinité tout en minimisant la liaison des autres molécules sur le support d'affinité. Typiquement, l'étape a) est réalisée en présence d'une solution tampon (appelée dans la suite « tampon de liaison »). Le tampon de liaison peut être mélangé avec la composition de départ avant l'étape a) ou il peut être ajouté pendant l'étape a). Le tampon de liaison est typiquement une solution aqueuse contenant un agent tampon. L'agent tampon peut être choisi de façon à être compatible avec le fibrinogène et le support d'affinité et de façon à donner le pH souhaité pour l'étape a). Par exemple, pour obtenir un pH d'environ 6,3, l'agent tampon peut être choisi, sans s'y limiter, parmi l'acide 3-(N-morpholino)propanesulfonique (MOPS), l'acide 2-(N-morpholino)éthanesulfonique (MES), l'HEPES, le Bis-TRIS, le citrate et l'acétate. L'agent tampon peut être présent en une concentration d'environ 5 mM à 1 mM, par exemple de 10 mM à 500 mM, par exemple de 10 mM à 200 mM, comme d'environ 50 mM.

Sans vouloir se lier à une quelconque théorie, la présence de sels peut favoriser la formation du complexe entre le fibrinogène et les groupements d'aptamère du support solide et/ou empêcher la liaison des autres molécules présentes dans la composition de départ. Typiquement, l'étape a) peut être réalisée en présence de chlorure de sodium, par exemple en une concentration s'échelonnant de 10 mM à 500 mM, de préférence de 50 mM à 350 mM, ou de 100 mM à 200 mM, comme d'environ 150 mM.

La présence de cations divalents peut moduler la liaison du fibrinogène aux groupements d'aptamère. Dans certains modes de réalisation, l'étape a) est réalisée en présence de cations divalents, tels que Mg2+, en une concentration d'au moins 1 mM, par exemple en une concentration d'environ 1 mM à 50 mM, par exemple de 1 mM à 20 mM, comme une concentration d'environ 5 mM.

Dans certains autres modes de réalisation, l'étape a) est réalisée en l'absence de Mg2+ ; et plus généralement, en l'absence de cations divalents.

Par conséquent, le tampon de liaison utilisé dans l'étape a) peut comprendre du NaCl en une concentration de 100 mM à 500 mM et un sel de magnésium tel que le chlorure de magnésium (MgCl2) en une concentration d'environ 1 mM à 50 mM, et il peut avoir un pH de 5,0 ou de 6,9. Ce tampon peut convenir lorsque les groupements d'aptamère présents sur le support solide sont choisis dans le premier sous-groupe de l'invention défini ci-dessus.

Un tampon de liaison approprié pour réaliser l'étape a), en particulier lorsque le groupement d'aptamère appartient au premier sous-groupe défini ci-dessus, peut être un tampon comprenant 50 mM de MOPS, 5 mM de MgCl₂ et 150 mM de NaCl, à pH 6,3.

Lorsque les groupements d'aptamère présents sur le support d'affinité sont choisis dans le second sous-groupe d'aptamères défini ci-dessus, le tampon de liaison peut être dépourvu de Mg2+, et plus généralement de cations divalents. Un tampon de liaison approprié pour réaliser l'étape a) peut donc être un tampon comprenant 50 mM de MOPS et 150 mM de NaCl, à pH 6,3.

A la fin de l'étape a), et avant l'étape b), le support d'affinité peut être lavé avec un tampon de lavage approprié de façon à éliminer les substances qui ne sont pas spécifiquement liées, mais adsorbées sur le support. Il va sans dire que le tampon de lavage n'altère pas significativement le complexe entre le fibrinogène et le groupement d'aptamère tout en favorisant une désorption des substances qui ne se lient pas spécifiquement au support d'affinité.

Ainsi, dans certains modes de réalisation, le procédé de l'invention comprend une étape de lavage du support d'affinité à la fin de l'étape a) et avant l'étape b). Tout tampon de lavage classique, bien connu de l'homme de métier, peut être utilisé. Dans certains modes de réalisation, le tampon de lavage a la même composition que celle du tampon de liaison utilisé dans l'étape a). Dans d'autres modes de réalisation, le tampon de lavage peut comprendre les mêmes composants, mais en des concentrations différentes, par rapport au tampon de liaison utilisé dans l'étape a). Dans des modes de réalisation supplémentaires ou alternatifs, le pH du tampon de lavage est le même que celui du tampon de liaison.

Le tampon de lavage peut avoir un pH inférieur à 7, par exemple de pH 5,0 à 6,9, de préférence de 6,1 à 6,5, comme pH 6,3. Le tampon de lavage peut en outre comprendre du NaCl. Typiquement, la force ionique du tampon de lavage peut être supérieure à celle du tampon de liaison. En fait, la demanderesse a montré que, pour certains aptamères de l'invention, une force ionique élevée peut ne pas altérer significativement la liaison du fibrinogène aux groupements d'aptamère. En d'autres termes, le complexe entre le fibrinogène et certains aptamères utilisés selon l'invention peut être stable, même en présence d'une force ionique élevée. Ainsi, dans certains modes de réalisation, la solution de lavage a une force ionique supérieure à celle du tampon de liaison utilisé dans l'étape a). Dans des modes de réalisation alternatifs ou supplémentaires, le tampon de lavage peut comprendre une concentration de NaCl d'au moins 100 mM et jusqu'à 10 M. Par exemple, la concentration du NaCl peut être d'environ 100 mM à 5 mM, de préférence de 150 mM à 2 mM. Éventuellement, le tampon de lavage comprend en outre des cations divalents, en particulier Mg2+, en une concentration d'environ 0,1 mM à 20 mM, de préférence de 1 mM à 10 mM, comme une concentration d'environ 5 mM. Dans certains modes de réalisation, le tampon de lavage est dépourvu de Mg2+ et plus généralement de cations divalents.

Dans certains autres modes de réalisation supplémentaires, le tampon de lavage peut comprendre au moins un composant supplémentaire, de préférence choisi parmi les alkyldiols, en particulier parmi l'éthylène glycol ou le propylène glycol. En fait, pour certains aptamères utilisé selon l'invention, la présence d'alkyldiols tels que l'éthylène glycol dans la solution de lavage n'altère pas le complexe entre le fibrinogène et l'aptamère. Le tampon de lavage peut donc comprendre un alkyldiol tel que l'éthylène glycol ou le propylène glycol en une quantité de 1 % à 70 % en poids, de préférence de 10 % à 60 % en poids, comme 50 % en poids.

À titre d'illustration uniquement, le tampon de lavage comprend du MOPS 50 mM, du NaCl 2M à pH 6,3 et éventuellement 50 % en poids de glycol. Ce tampon de lavage peut convenir à la réalisation du lavage d'un support d'affinité portant des groupements d'aptamère appartenant au second sous-groupe d'aptamères décrit ci-dessus. Un autre exemple de tampon de lavage est une solution comprenant du MOPS 50 mM, du NaCl 0,5M et du MgCl₂ 5 mM à pH 6,3.

L'étape b) a pour but de libérer le fibrinogène du complexe formé dans l'étape a). Cette libération peut être obtenue par déstabilisation du complexe entre le fibrinogène et les groupements d'aptamère, à savoir à l'aide de conditions qui réduisent l'affinité des aptamères pour le fibrinogène. Il convient de noter que le complexe entre le groupement d'aptamère et le fibrinogène peut être déstabilisé dans de conditions modérées qui ne sont pas susceptibles d'altérer le fibrinogène.

Comme expliqué ci-dessus, la capacité des aptamères utilisés selon l'invention à se lier au fibrinogène peut dépendre du pH du milieu. Une augmentation du pH au-delà de 7,0 peut permettre de favoriser la libération du fibrinogène. Ainsi, dans certains modes de réalisation, l'étape b) est réalisée par augmentation du Ph au-delà de 7,0. De préférence, le pH de l'étape b) est de 7,0 à 8,0, par exemple de 7,2 à 7,8, comme un pH de 7,4. En d'autres termes, un tampon d'élution à un pH supérieur à 7,0 peut être utilisé pour favoriser la libération du fibrinogène. À titre d'illustration uniquement, un tampon d'élution approprié peut être une solution tamponnée de MOPS 50 mM à pH 7,4 et comprenant du NaCl 150 mM.

Comme expliqué ci-dessus, la capacité de l'aptamère à se lier au fibrinogène peut aussi varier en fonction de la présence de cations divalents, tels que Mg2+. Par exemple, la liaison du groupement d'aptamère au fibrinogène peut être favorisée par la présence de Mg2+. Ainsi, la libération du fibrinogène du complexe dans l'étape b) peut être favorisée à l'aide d'un tampon d'élution dépourvu de cations divalents et/ou comprenant un agent chélatant les cations divalents, tel que l'EDTA ou l'EGTA. Par exemple, l'agent chélatant les cations divalents peut être présent en une concentration d'au moins 1 mM et d'au plus 500 mM dans le tampon d'élution utilisé dans l'étape b). L'utilisation d'un agent chélatant les cations divalents peut être appropriée pour un support d'affinité portant des aptamères appartenant au premier sous-groupe décrit ci-dessus.

Dans d'autres modes de réalisation, la liaison du groupement d'aptamère au fibrinogène peut diminuer en présence de cations divalents tels que Mg2+. Ainsi, dans ce mode de réalisation, le tampon d'élution peut comprendre des cations divalents, en particulier Mg2+, en une concentration d'environ 0,1 mM à 20 mM, de préférence de 1 mM à 10 mM, comme une concentration d'environ 5 mM. Cette élution peut convenir à la libération du fibrinogène du complexe formé avec un groupement d'aptamère appartenant au second sous-groupe défini ci-dessus.

Un autre exemple de tampon d'élution qui peut être utilisé dans l'étape b) est une solution de MOPS 50 mM à pH 7,4 comprenant du MgCl₂ 2 M.

À la fin de l'étape c), le fibrinogène purifié est typiquement obtenu sous la forme d'une composition liquide purifiée. Cette composition liquide purifiée peut subir une ou plusieurs étapes supplémentaires. Ladite composition liquide peut être concentrée et/ou soumise à une inactivation ou une élimination virale, par exemple par une filtration stérile ou par un détergent, une diafiltration, une étape de formulation avec un ou plusieurs excipients pharmaceutiquement acceptables, une lyophilisation, un emballage, de préférence dans des conditions stériles, et des combinaisons de ceux-ci.

Dans un aspect plus général, le procédé de purification du fibrinogène ou le procédé de préparation d'une composition de fibrinogène purifiée peut comprendre une ou plusieurs étapes supplémentaires y compris, sans s'y limiter, une ou des étape(s) de chromatographie telles qu'une chromatographie d'exclusion, une chromatographie d'échange d'ions, une chromatographie multimodale, une chromatographie à phase inversée, une chromatographie sur hydroxyapatite ou une chromatographie d'affinité, une étape de précipitation, une ou plusieurs étapes de filtration telles qu'une filtration poussée, une ultrafiltration, une ultrafiltration tangentielle, une nanofiltration, et une osmose inverse, une étape de clarification, une étape d'inactivation ou d'élimination virale, une stérilisation, une formulation, une lyophilisation, un emballage et des combinaisons de ceux-ci.

Dans certains modes de réalisation supplémentaires, le procédé de purification du fibrinogène ou le procédé de préparation d'une composition de fibrinogène purifiée comprend l'une des combinaisons de caractéristiques suivantes :
- combinaison 1 : (i) le groupement d'aptamère est choisi parmi les aptamères du premier sous-groupe défini ci-dessus, (ii) l'étape (a) est réalisée à pH 5,8 à 6,5, de préférence 6,3, en présence de Mg2+ et (iii) l'étape (b) est réalisée à pH 7,0 à 8,0, de préférence 7,4, éventuellement en présence d'un agent chélatant les cations divalents, et
- combinaison 2 : (i) le groupement d'aptamère est choisi parmi les aptamères du second sous-groupe défini ci-dessus, (ii) l'étape (a) est réalisée à pH 5,8 à 6,5, de préférence 6,3, en l'absence de Mg2+ et (iii) l'étape (b) est réalisée à pH 7,0 à 8,0, de préférence 7,4, en présence de Mg2+.

Dans un aspect supplémentaire, les aptamères et les ligands d'affinité de l'invention peuvent être utilisés dans un procédé de fractionnement du plasma sanguin. Le procédé de fractionnement du plasma sanguin peut comprendre plusieurs étapes de chromatographie d'affinité successives, chaque étape de chromatographie d'affinité permettant de récupérer une protéine plasmatique d'intérêt telle que le fibrinogène , une immunoglobuline, une albumine et d'autres facteurs de coagulation, tels que les facteurs de coagulation dépendant de la vitamine K. Les ligands d'affinité utilisés dans chaque étape peuvent être d'un type quelconque, en particulier des aptamères. À cet égard, la demanderesse a montré de façon surprenante que les protéines plasmatiques telles que le fibrinogène, l'albumine et l'immunoglobuline, peuvent être récupérées et purifiées à partir de plasma sanguin en réalisant des étapes de chromatographie d'affinité à base d'aptamères successives. Il convient de noter que le procédé de fractionnement du plasma sanguin comprenant des étapes de chromatographie d'affinité à base d'aptamères successives permet d'obtenir un concentré de fibrinogène et un concentré d'immunoglobuline avec une pureté de protéine d'au moins 96 %, et même d'au moins 99 %, avec des rendements d'environ 9-12 g par litre de plasma pour les immunoglobulines et de 2-4 g par litre de plasma pour le fibrinogène. La demanderesse a en outre montré que ces bons rendements et ces bons taux de pureté peuvent être atteints à partir de plasma sanguin brut. En d'autres termes, les étapes de chromatographie d'affinité à base d'aptamères peuvent être réalisées sur un plasma sanguin brut sans aucun prétraitement tel qu'un fractionnement à l'éthanol (procédé de Cohn), une cryo-précipitation, un fractionnement au caprylate ou une précipitation au PEG. Il convient de noter que ce procédé de fractionnement permet d'éviter des stockages à froid intermédiaires temporaires.

Un autre objet de l'invention est donc un procédé de fractionnement de plasma sanguin comprenant :
(a) une étape de chromatographie d'affinité pour récupérer le fibrinogène dans laquelle le ligand d'affinité est un aptamère qui se lie spécifiquement au fibrinogène, et
(b) une étape de chromatographie d'affinité pour récupérer les immunoglobulines (Ig) dans laquelle le ligand d'affinité est un aptamère qui se lie spécifiquement aux immunoglobulines,
dans lequel les étapes de chromatographie d'affinité (a) et (b) peuvent être réalisées dans un ordre quelconque.

De préférence, des immunoglobulines d'isotope G sont récupérées dans l'étape (b).

L'étape de chromatographie d'affinité pour récupérer le fibrinogène peut être réalisée avant la chromatographie d'affinité pour récupérer l'Ig et inversement. Par conséquent, dans certains modes de réalisation, le procédé de fractionnement de plasma sanguin comprend les étapes de :
- soumission de plasma sanguin ou d'un dérivé de celui-ci à une étape de chromatographie d'affinité, dans laquelle le ligand d'affinité est un aptamère qui se lie spécifiquement au fibrinogène , et
- soumission de la fraction non retenue, qui est substantiellement dépourvue de fibrinogène, à une étape de chromatographie d'affinité, dans laquelle le ligand d'affinité est un aptamère qui se lie spécifiquement à de l'immunoglobuline.

Il va sans dire que les étapes ci-dessus peuvent comprendre la récupération du fibrinogène et d'Ig retenus sur le support d'affinité, respectivement.

Dans certains autres modes de réalisation, le procédé de fractionnement de plasma sanguin comprend les étapes de :
- soumission de plasma sanguin ou d'un dérivé de celui-ci à une étape de chromatographie d'affinité, dans laquelle le ligand d'affinité est un aptamère qui se lie spécifiquement à une Ig, et
- soumission de la fraction non retenue, qui est substantiellement dépourvue d'Ig, à une étape de chromatographie d'affinité, dans laquelle le ligand d'affinité est un aptamère qui se lie spécifiquement à du fibrinogène.

Il va sans dire que les étapes ci-dessus peuvent comprendre la récupération d'Ig et du fibrinogène retenus sur le support d'affinité, respectivement.

Dans le procédé de l'invention, la composition de départ peut être un plasma sanguin ou des dérivés de celui-ci. Les dérivés de plasma sanguin englobent, sans s'y limiter, un plasma sanguin clarifié, un plasma sanguin appauvri en lipides, un cryo-précipité de plasma sanguin, un surnagent d'un cryo-précipité de plasma sanguin, une fraction de plasma, et analogues. Dans certains modes de réalisation, la composition de départ est un plasma sanguin brut.

De préférence, des immunoglobulines de l'isotope G sont récupérées. Les immunoglobulines de l'isotope G englobent l'IgG1, l'IgG2, l'IgG3 et l'IgG4. Dans certains modes de réalisation, l'aptamère dirigé contre l'immunoglobuline est capable de se lier spécifiquement à l'IgG, indépendamment des sous-catégories d'IgG. Dans certains modes de réalisation, plusieurs types d'aptamères anti-IgG sont utilisés pour récupérer toutes les sous-catégories d'IgG. De préférence, la fraction d'IgG récupérée dans le procédé de fractionnement de l'invention a une répartition des sous-catégories proche de celle du plasma sanguin de départ, c'est-à-dire qu'elle comprend de 50 % à 70 % d'IgG1, de 25 % à 35 % d'IgG2, de 2 % à 8 % d'IgG3 et de 1 % à 8 % d'IgG4.

Dans certains modes de réalisation, le procédé de fractionnement de plasma sanguin de l'invention comprend une ou plusieurs étapes supplémentaires, en particulier (c) une étape de purification de l'albumine.

L'albumine purifiée peut être récupérée par un quelconque procédé classique tel qu'une chromatographie y compris une chromatographie d'affinité, une chromatographie d'échange d'ions et une précipitation à l'éthanol suivie d'une filtration.

Par exemple, l'étape (c) peut être une étape de chromatographie d'affinité dans laquelle le ligand d'affinité est un aptamère qui se lie spécifiquement à l'albumine.

Lorsque l'étape (c) est présente, les étapes (a), (b) et (c) peuvent être réalisées dans un ordre quelconque. Dans certains modes de réalisation, l'étape (c) est réalisée sur la fraction non retenue obtenue de l'étape (a) ou de l'étape (b).

Un quelconque type de technologie de chromatographie peut être utilisé pour réaliser les étapes (a), (b) et (c) dans le procédé de l'invention, tel qu'une chromatographie discontinue, une chromatographie sur lit mobile simulé (SMB). Les technologies de chromatographie préférées sont celles comprenant l'utilisation de plusieurs colonnes telles que la chromatographie sur SMB.

Le procédé de fractionnement de plasma sanguin peut comprendre une ou plusieurs étapes supplémentaires y compris, sans s'y limiter, une étape de chromatographie pour éliminer les anticorps anti-A et/ou anti-B, une ultrafiltration, une ultrafiltration tangentielle, une nanofiltration, une osmose inverse, une clarification, une étape d'inactivation virale, une étape d'élimination virale, une stérilisation, des étapes de polissage telles qu'une formulation, ou une lyophilisation ou des combinaisons de ceux-ci. Le procédé de l'invention peut aussi comprendre une ou plusieurs étapes supplémentaires dans le but de prévenir et/ou d'éliminer l'encrassement des colonnes de chromatographie telles qu'un traitement sanitaire avec une solution alcaline, par exemple avec une solution d'hydroxyde de sodium.

L'invention concerne aussi une composition de fibrinogène purifiée susceptible d'être obtenue ou obtenue par un procédé de préparation d'une composition de fibrinogène purifiée selon l'invention ou par le procédé de fractionnement de plasma sanguin selon l'invention.

Un autre objet de l'invention est une composition de fibrinogène purifiée qui comprend au moins 90 % en poids, de préférence au moins 91 %, 92 %, 93 %, 94 %, 95 %, 96 %, 97 %, 98 %, 99 %, 99,1 %, 99,2 %, 99,3 %, 99,4 %, 99,5 %, 99,5 %, 99,6 %, 99,7 %, 99,8 % ou 99,9 % en poids, par rapport au poids total des protéines présentes dans ladite composition. Dans certains modes de réalisation, la composition de fibrinogène purifiée comprend du fibrinogène plasmatique humain, par exemple le fibrinogène obtenu à partir de plasma humain. Dans ce mode de réalisation, ladite composition comprend au plus 10 %, de préférence au plus 9 %, 8 %, 7 %, 6 %, 5 %, 4 %, 3 %, 2 %, 1 %, 0,9 %, 0,8 %, 0,7 %, 0,6 %, 0,5 %, 0,4 %, 0,3 %, 0,2 % ou 0,1 % en poids d'autres protéines plasmatiques, en particulier d'autres facteurs de coagulation humains. Dans certains modes de réalisation, la composition est substantiellement dépourvue de facteurs de coagulation humains autres que le fibrinogène humain. Dans certains modes de réalisation supplémentaires ou alternatifs, ladite composition est dépourvue de facteur XIII.

Dans certains modes de réalisation, la composition de fibrinogène purifiée comprend le fibrinogène recombinant humain, par exemple le fibrinogène humain produit par un hôte recombinant tel qu'une cellule recombinante ou un animal transgénique. Dans ce mode de réalisation, ladite composition comprend au plus 10 %, de préférence au plus 9 %, 8 %, 7 %, 6 %, 5 %, 4 %, 3 %, 2 %, 1 %, 0,9 %, 0,8 %, 0,7 %, 0,6 %, 0,5 %, 0,4 %, 0,3 %, 0,2 % ou 0,1 % en poids d'autres protéines, en particulier d'autres protéines non humaines provenant de l'hôte recombinant. Dans certains modes de réalisation, la composition est substantiellement dépourvue de protéines non humaines. Dans certains modes de réalisation supplémentaires ou alternatifs, ladite composition est dépourvue de tout homologue non humain de fibrinogène qui peut être trouvé dans l'hôte recombinant.

L'invention concerne aussi une composition pharmaceutique comprenant une composition purifiée du fibrinogène humain tel que le fibrinogène humain recombinant ou le fibrinogène plasmatique humain tel que défini ci-dessus, en combinaison avec un ou plusieurs excipients pharmaceutiquement acceptables. Ladite composition pharmaceutique, ainsi que la composition liquide purifiée de fibrinogène selon l'invention, peut être utilisée dans le traitement de troubles de la coagulation, en particulier dans les traitements d'une déficience congénitale ou acquise de fibrinogène (l'hypo-, la dys- ou l'a-fibrinogémie). La composition de l'invention peut être utilisée dans la gestion de saignements post-traumatiques ou post-chirurgicaux aigues ou dans la gestion d'une déficience en fibrinogène résultant d'une défaillance rénale aigues.

### Procédé d'obtention des aptamères utilisés selon l'invention

La demanderesse a réalisé plusieurs stratégies SELEX décrites dans l'art antérieur pour identifier des aptamères dirigés contre le fibrinogène humain. Aucune de ces stratégies n'a réussi et un SELEX standard a conduit à l'identification d'aptamères dirigés contre un contaminant, ce qui explique pourquoi moins de 1 % de la composition de fibrinogène purifiée est utilisé pour le réaliser.

Dans ce contexte, la demanderesse a réalisé des recherches poussées pour développer un nouveau procédé d'obtention d'aptamères dirigés contre des protéines « SELEX-résistantes » telles que le fibrinogène.

La demanderesse a conçu un nouveau procédé SELEX qui permet d'obtenir des aptamères présentant une forte affinité de liaison pour des protéines « SELEX-résistantes », et qui peut être utilisé comme ligands d'affinité dans un procédé de purification. Ce nouveau procédé SELEX est caractérisé par une étape de sélection qui est réalisée dans des conditions de pH convenant à la création de « patchs positifs » à la surface de la protéine cible. En d'autres termes, le procédé conçu par la demanderesse est basé sur le renfort des interactions locales entre les aptamères potentiels et la protéine cible en favorisant des charges positives sur un domaine superficiel de la protéine. Ce procédé peut être réalisé pour des protéines ayant une ou plusieurs histidines superficielles, telles que le fibrinogène. Le pH de l'étape de sélection (à savoir l'étape dans laquelle la protéine cible est mise en contact avec le mélange candidat d'acides nucléiques) doit être choisi de façon à favoriser la protonation d'au moins une histidine superficielle de la protéine cible. Dans le cas du fibrinogène, la demanderesse a montré qu'un pH approprié pour l'étape de sélection est un pH légèrement acide.

Par conséquent, un procédé d'obtention d'un aptamère utilisé selon l'invention qui se lie spécifiquement au fibrinogène, comprend les étapes suivantes :
a) la mise en contact du fibrinogène avec un mélange candidat d'acides nucléiques à un pH inférieur à 7,0, de préférence de 5,8 à 6,8,
b) la récupération des acides nucléiques qui se lient au fibrinogène, tout en éliminant les acides nucléiques non liés,
c) l'amplification des acides nucléiques obtenus dans l'étape (b) pour produire un mélange candidat d'acides nucléiques ayant une affinité accrue pour le fibrinogène, et
d) la répétition des étapes (a), (b), (c) jusqu'à l'obtention d'un ou de plusieurs aptamères dirigés contre le fibrinogène.

Dans l'étape (a), le mélange candidat d'acides nucléiques est généralement un mélange d'acides nucléiques statistiques synthétisés chimiquement. Le mélange candidat peut comprendre de 108 à 1018, typiquement environ 1015 acides nucléiques. Le mélange candidat peut être un mélange d'acides nucléiques d'ADN ou mélange d'acides nucléiques d'ARN. Dans certains modes de réalisation, le mélange candidat est constitué d'une multitude d'ADN monocaténaires (ADNss), dans laquelle chaque ADNss comprend une séquence statistique centrale d'environ 20 à 100 nucléotides flanquée de séquences spécifiques d'environ 15 à 40 nucléotides qui jouent le rôle d'amorces pour une amplification par PCR. Dans certains autres modes de réalisation, le mélange candidat est constitué d'une multitude d'acides nucléique d'ARN, dans laquelle chaque ARN comprend une séquence statistique centrale d'environ 20 à 100 nucléotides flanquée de séquences d'amorce d'environ 15 à 40 nucléotides pour une amplification par RT-PCR. Dans certains modes de réalisation, le mélange candidat d'acides nucléiques est constitué d'acides nucléiques non modifiés, ce qui signifie que les acides nucléiques comprennent uniquement des nucléotides existant naturellement. Dans certains autres modes de réalisation, le mélange candidat peut comprendre des acides nucléiques chimiquement modifiés. En d'autres termes, les acides nucléiques peuvent comprendre un ou plusieurs nucléotides chimiquement modifiés. Dans des modes de réalisation préférés, le mélange candidat est constitué d'ADN monocaténaires.

L'étape a) est réalisée dans des conditions favorables à la liaison du fibrinogène à des acides nucléiques ayant une affinité pour ledit fibrinogène. De préférence, le pH de l'étape a) est de 6,0 à 6,6, comme 6,1, 6,2, 6,3, 6,4 et 6,5. Un pH approprié pour l'étape a) est, par exemple, de 6,3 ± 0,1. Ce pH permet de protoner au moins une histidine superficielle du fibrinogène. L'étape (a) peut être réalisée dans une solution aqueuse tamponnée. L'agent tampon peut être choisi parmi un quelconque agent tampon permettant d'obtenir le pH souhaité et compatible avec les protéines cibles et le mélange d'acides nucléiques. L'agent tampon peut être choisi, sans s'y limiter, parmi l'acide 3-(N-morpholino)propanesulfonique (MOPS), l'acide 2-(N-morpholino)éthane-sulfonique (MES), l'HEPES, le Bis-Tris, le citrate et l'acétate. L'agent tampon peut être présent en une concentration d'environ 5 mM à 1 M, par exemple de 10 mM à 500 mM, par exemple de 10 mM à 200 mM, comme d'environ 50 mM.

Dans un mode de réalisation particulier de l'invention, la composition liquide, stable et comprenant du fibrinogène est obtenue selon le procédé comprenant les étapes suivantes :
- obtention d'une fraction de cryosurnageant de plasma sanguin,
- précipitation du cryo-surnageant par 8 % d'éthanol afin d'obtenir une fraction enrichie en fibrinogène,
- purification de la fraction de plasma sanguin enrichie en fibrinogène après remise en suspension par séparation sur gel de chromatographie d'affinité utilisant préférentiellement des ligands d'affinité choisis parmi les anticorps, les fragments d'anticorps, les dérivés d'anticorps ou des ligands chimiques tels que des peptides, des peptides mimétiques, des peptoïdes, des nanofitines ou encore des ligands oligonucléotidiques tels que les aptamères,
- récupération de la fraction adsorbée purifiée comprenant du fibrinogène,
- éventuellement, ajout d'excipients pharmaceutiquement acceptables, de préférence de l'arginine et/ou du citrate.

Dans un mode de réalisation particulier, le procédé comprend en outre une étape de conservation pendant au moins 3 mois à 4°C.

Dans un mode de réalisation particulier de l'invention, la chromatographie d'affinité utilisée est une matrice d'affinité avec des ligands de type fragment dérivé d'anticorps de lama telle que la matrice Fibrinogen CaptureSelect (Life Technologies).

De manière particulièrement avantageuse, la composition selon l'invention est dépourvue de protéases et/ou d'activateurs de la fibrinolyse.

Par « composition de fibrinogène dépourvue de protéases et/ou d'activateurs de la fibrinolyse», il est entendu que la composition de fibrinogène a subi une ou plusieurs étapes permettant l'élimination des protéases telles que la thrombine, la prothrombine, plasmine, plasminogène de telle manière que la quantité résiduelle de protéases et/ou d'activateurs de la fibrinolyse soit :
- très fortement réduite en comparaison à la solution de Fibrinogène pré-purifiée avant étape de chromatographie, et/ou
- nulle, et/ou
- inférieure aux seuils de détection des méthodes communément utilisées par l'homme du métier.

De manière avantageuse, le taux de prothrombine résiduel est inférieur à 5 µUI / mg de fibrinogène, le taux de plasminogène est inférieur à 15 ng / mg de fibrinogène.

Dans un mode de réalisation particulier de l'invention, la composition selon l'invention est donc dépourvue de protéases telles que la thrombine et/ou la plasmine ou leurs pro-enzymes correspondants prothrombine (facteur II de la coagulation) et/ou plasminogène, qui sont des zymogènes potentiellement activables.

Dans un mode de réalisation particulier de l'invention, la composition de fibrinogène selon l'invention est dépourvue d'inhibiteurs de protéases et/ou d'anti-fibrinolytiques.

Par « inhibiteurs de protéases et/ou anti-fibrinolytiques», on entend toute molécule à activité antiprotéasique, notamment toute molécule à activité inhibitrice de sérine protéase et/ou anti-fibrinolytique, en particulier toute molécule à activité inhibitrice de thrombine et/ou antiplasmine, en particulier l'hirudine, la benzamidine, l'aprotinine, le florure de phénylméthylsulfonyle (PMSF), la pepstatine, la leupeptine, l'antithrombine III associée ou non avec del'héparine, l'alpha 2 macroglobuline, l'alpha 1 antitrypsine, l'acide hexanoïque ou epsilon aminocaproïque, l'acide tranéxamique, l'alpha 2 antiplasmine.

Dans un mode de réalisation particulier de l'invention, la composition de fibrinogène selon l'invention est dépourvue d'hirudine et/ou de benzamidine et/ou d'aprotinine et/ou de PMSF et/ou de pepstatine et/ou de leupeptine et/ou d'antithrombine III associée ou non avec de l'héparine et/ou d'alpha 2 macroglobuline et/ou d'alpha 1 antitrypsine et/ou d'acide hexanoïque et/ou epsilon aminocaproïque et/ou d'acide tranéxamique et/ou d'alpha 2 antiplasmine.

Dans un mode de réalisation particulier de l'invention, la composition de fibrinogène selon l'invention est dépourvue d'ions métalliques.

Dans un mode de réalisation particulier de l'invention, la composition selon l'invention est avantageusement dépourvue de calcium.

Dans un mode de réalisation particulier de l'invention, la composition de fibrinogène selon l'invention est dépourvue d'isoleucine, de glycine et/ou de NaCl.

Dans un mode de réalisation particulier de l'invention, la composition de fibrinogène selon l'invention est dépourvue d'albumine.

De manière avantageuse, la composition selon l'invention possède une pureté supérieure ou égale à 70%, de manière préférée supérieure ou égale à 75%, 80%, 85%, 90%, 95%, 96%, 97%, 98%, 99%.

Dans un mode de réalisation particulier de l'invention, la composition selon l'invention ne comprend pas d'autres protéines copurifiées, avantageusement pas de FXIII et/ou de fibronectine.

Dans un autre mode de réalisation particulier de l'invention, la composition de fibrinogène selon l'invention peut également comprendre une ou plusieurs protéines accompagnantes, éventuellement copurifiées. Dans un mode de réalisation particulier de l'invention, la composition selon l'invention comprend avantageusement du FXIII.

La composition selon l'invention est soumise, directement après purification, aux étapes de mise en forme pharmaceutique sous forme liquide : formulation, filtration stérilisante et répartition en contenant (flacon ou autre dispositif de conservation/administration).

De manière particulièrement avantageuse, la composition selon l'invention n'est soumise à aucune étape de lyophilisation, dessiccation, déshydratation ou séchage.

De manière particulièrement avantageuse, la composition selon l'invention est donc sous forme liquide sans avoir subi d'étape de reconstitution d'un lyophilisat.

De manière particulièrement avantageuse, la composition selon l'invention est sous forme liquide, et comprend donc, outre les éventuels excipients pharmaceutiquement acceptables, de l'eau.

Dans un mode de réalisation particulier de l'invention, la composition sous forme liquide comprend du citrate et/ou de l'arginine, de préférence moins de 300mM d'arginine.

De manière avantageuse, la composition selon l'invention est particulièrement adaptée à une administration par voie intraveineuse.

Un autre aspect de l'invention concerne la composition selon l'invention pour son utilisation en thérapie. Toutes les caractéristiques techniques énoncées précédemment s'appliquent ici.

L'exemple qui suit illustre un mode de réalisation de la présente invention sans toutefois en limiter la portée.

### Exemple 1 : Composition de fibrinogène liquide stable

### Matériel & méthode

### Fraction plasmatique

La matière première de départ est un pool de plasma humain préalablement soumis à une étape de cryo-précipitation puis soumis à une étape de précipitation avec 8% d'éthanol.

La solution de fibrinogène plasmatique pré-purifiée ainsi obtenue est diluée par 2 volumes de tampon d'équilibration du gel avant injection sur la colonne de chromatographie pré-équilibrée en pH et en conductivité.

Caractéristiques de la fraction plasmatique : fibrinogène antigénique ajusté par dilution à 4,7-4,8 g/L.

### Solutions tampon

La composition des solutions tampon utilisées lors des différentes étapes du procédé de chromatographie est résumée dans le tableau 1 ci-dessous.

**Tableau 1 : Solutions tampon pour la chromatographie d'affinité.**

| Phases | Composition | Valeurs cibles |
|---|---|---|
| Equilibration de la colonne et retour à la ligne de base | Citrate tri-sodique 10 mM, Arginine HCl 0,1 M | pH ajusté à 7,4 |
| Pré-élution | Citrate tri-sodique 10 mM, Chlorure de Sodium 2,0 M | pH ajusté à 7,4 |
| | Arginine HCl 0,1 M | |
| Elution | Citrate tri-sodique 10 mM, Propylène glycol 50 % v/v | pH ajusté à 7,4 |
| | Arginine HCl 1,0 M | |

### Gels de chromatographie d'affinité

Pour la chromatographie, on utilise un gel d'affinité CaptureSelect Fibrinogen (Life Technologies ref. 191291050, lots 171013-01 et 171013-05), présentant un ligand d'affinité constitué d'un fragment d'anticorps de lama anti-fibrinogène.

### Colonnes

Une colonne de 50 mm de diamètre (Référence XK 50/30 GE Healthcare), volume de gel packé de 67 ml ; pour une hauteur de colonne de 3,4 cm a été utilisée

### Purification par affinité

La solution de fibrinogène ajustée à environ 5 g/L est injectée sans autre ajustement sur la colonne d'affinité CaptureSelect Fibrinogène équilibrée. Une charge d'environ 10 g/L a été appliquée.

L'éluat de chromatographie a ensuite été ultrafiltré et pré-formulé sur membrane de seuil de coupure 100 kDa (référence Pall Omega OS 100C 10)
A la fin du procédé, le produit obtenu a une concentration en fibrinogène coagulable de 15,2 g/L et en fibrinogène antigénique de 15,2 mg/ml.

### Exemple 2 : stabilité d'une composition de fibrinogène liquide

### Composition de fibrinogène

La composition de fibrinogène obtenue selon la méthode illustrée à l'exemple 1 est formulée à minima avec de l'arginine 100 mM et du citrate 8,5 mM à pH 7,0.

### Stabilité de la composition de fibrinogène

La composition est mise en stabilité, sous air (à l'air libre), sans agitation, en chambre froide à +5°C.

Des échantillons sont prélevés avant la mise en stabilité (T0) et à différents temps suivant le début de la mise en stabilité : deux mois (T2M) et 3 mois (T3M).

### Résultats de la mise en stabilité à 5°C

Les résultats de cet essai de mise en stabilité sont présentés dans le tableau ci-dessous.

| Fibrinogène liquide | | **T0** | **T2M** | **T3M** |
|---|---|---|---|---|
| Formulation minimale | | | | |
| Mise en stabilité à 5°C | | | | |
| Concentration (DO à 280 nm) | | 18,5 | 16,8 | 18,5 |
| pH | | 6,9 | 6,9 | 6,9 |
| Osmolalité | | 209 | 207 | 210 |
| Fibrinogène coagulable | | 20 | 15,7 | 15,7 |
| Fibrinogène antigénique | | 16,5 | 15,6 | 16,2 |
| Ratio fibrinogène coagulable/antigènique | | 1,2 | 1,0 | 1,0 |
| SDS PAGE en conditions réduites | Aα1 (64 kDa) | 25,9 | 23,1 | 23,2 |
| | Aα2 (62 kDa) | 10,7 | 10,1 | 9,5 |
| | Aα3 (60 kDa) | 3,9 | 5,2 | 5,0 |
| | ∑Aαₙ | 40,5 | 38,4 | 37,7 |
| | Bβ (54 kDa) | 30,8 | 31,1 | 31,2 |
| | γ' (50 kDa) | 4,7 | 4,0 | 3,1 |
| | γ (48 kDa) | 23,9 | 26,5 | 27,9 |
| | Σγ | 28,6 | 30,5 | 31,0 |

Les résultats obtenus 2 et 3 mois après le début de la mise en stabilité montrent que :
- Les résultats de SDS PAGE en conditions réduites montrent une faible variation des profils des chaînes alpha, beta et gamma qui restent stables dans le temps, confirmant la conservation de l'intégrité du fibrinogène liquide ;
- Le ratio fibrinogène coagulable/fibrinogène antigène est conservé et stable à 1, confirmant la conservation de l'intégrité et de la fonctionnalité du fibrinogène liquide.

Les résultats de cet essai démontrent clairement que la composition de fibrinogène est stable sous forme liquide à 5°C pendant 3 mois.

### Exemple 3 : Identification d'aptamères anti-fibrinogène utilisés selon l'invention

### 1. Matériel et méthode

### Banques d'oligonucléotides

La banque d'ADNss utilisée pour réaliser le procédé SELEX est constituée d'une région aléatoire de 40 bases flanquée par deux régions d'amorce constantes de 18 bases.

### Fibrinogène

La protéine cible est le fibrinogène humain. Différentes sources de fibrinogène humain ont été utilisées pendant le procédé SELEX :
Fibrinogène humain : Deux préparations de fibrinogène humain ont été utilisées et préparée sous forme d'une composition purifiée à partir de plasma humain ayant une pureté respectivement de 95 % et de 99,9 %.
Fibrinogène transgénique : Le fibrinogène transgénique a été purifié à partir de lait de vaches transgéniques jusqu'à une pureté de 97 %.

### Protocole SELEX

Le fibrinogène (fibrinogène transgénique pur à 97 % pour les essais 1 à 3 et le fibrinogène plasmatique pur à 95 % pour les essais 4 et 5 et le fibrinogène plasmatique pur à 99,9 % pour les essais 6 à 8) a été immobilisé sur une résine d'affinité, alors que la quantité de cible immobilisée sur la résine a diminué de façon continu des essais 1 à 8 (voir la figure 8).

La cible immobilisée a été incubée avec une banque/pool d'ADNss à des concentrations décroissantes à l'aide d'un tampon de sélection (MOPS 50 mM, pH 6,30, NaCl 150 mM, MgCl₂ 5 mM) pendant un temps d'incubation décroissant (voir le tableau de la figure 8).

La résine contenant le fibrinogène/ADNss a été récupérée et lavée avec le tampon de sélection pendant les essais 1 et 2 et un tampon de lavage contenant du MOPS 50 mM, pH 6,30, NaCl 500 mM, MgCl₂ 5 mM des essais 3 à 8 (voir le tableau). Après le lavage, l'ADNss lié a été élué à l'aide d'un tampon d'élution (Tris-HCl 50 mM, pH 7,40, EDTA 200 mM). Avant chaque essai (sauf le premier essai), une étape de sélection de contre-ions a été réalisée par incubation de l'ensemble d'ADNss avec la résine d'affinité afin d'empêcher l'enrichissement des aptamères anti-support. Les paramètres des protocoles SELEX sont décrits sur la figure 8.

### Détermination de l'affinité de liaison des aptamères par SPR

L'aptamère choisi a été synthétisé avec de la biotine et un spacer de triéthylène glycol à l'extrémité 5' de l'oligonucléotide. Une solution 1 µM de l'aptamère a été préparée à l'aide du tampon de sélection SELEX. La solution d'aptamère a été chauffée à 90 °C pendant 5 min, incubée dans de la glace pendant 5 min et équilibrée à la température ambiante pendant 10 min. La préparation a été injectée dans une puce de capteur revêtue de streptavidine SA de Biocore T200 instrument (GE Healthcare) à un débit de 10 µl/ml pendant 7 min. Ensuite, différentes concentrations de la cible ont été injectées sur l'aptamère immobilisé à raison de 30 µl/min pendant 1 minute. Après une dissociation pendant 1-2 min, une étape de lavage a été réalisée par injection d'un tampon de lavage convenable à raison de 30 µl/min pendant 1 min. Pour l'élution, un tampon d'élution convenable a été injecté à raison de 30 µl/min pendant 1-2 min. Finalement, la puce de capteur a été régénérée par injection de NaOH 50 mM à raison de 30 µl/min pendant 30 s. Au cours de l'expérience, le signal de réponse a été enregistré dans un sensogramme.

### 2. Résultats

Le procédé SELEX a permis d'identifier 67 candidats aptamères anti-fibrinogène, parmi lesquels des aptamères de SEQ ID N° 1, SEQ ID N° 58, SEQ ID N° 60 et 65 qui ont présenté une forte affinité à la fois pour le fibrinogène humain plasmatique et transgénique.

Ces aptamères s'avèrent se lier au fibrinogène humain d'une façon dépendante du pH. Les séquences de basedes aptamères de SEQ ID N° 1 et de SEQ ID N° 58 (à savoir la séquence minimale se liant au fibrinogène ) ont été déterminées. L'aptamère de SEQ ID N° 66 correspond à la séquence de base de l'aptamère de SEQ ID N° 1. L'aptamère de SEQ ID N° 67 correspond à la séquence de base de l'aptamère de SEQ ID N° 58. Les figures 3A-3D présentent le profil de liaison obtenu pour les séquences de base des aptamères de SEQ ID N° 1 et N° : 58 par SPR. Les aptamères de SEQ ID N° 66 et 67 sont capables de se lier spécifiquement au fibrinogène transgénique et au fibrinogène plasmatique à pH 6,3 d'une façon dépendante de la dose, comme le montre l'augmentation des signaux lorsque la concentration de fibrinogène augmente. Le complexe entre les aptamères et le fibrinogène n'était pas significativement dissocié par l'augmentation de la concentration de NaCl. D'autre part, l'injection d'un tampon à pH 7,4 a permis de dissocier le complexe entre les aptamères et, de ce fait, le fibrinogène a été élué (figures 3A-3D).

En fait, les aptamères obtenus pas le procédé de l'invention se lient au fibrinogène d'une façon dépendante du pH. Ce résultat est illustré sur les figures 4A et 4B respectivement pour des aptamères de SEQ ID N° 66 et SEQ ID N° 67. Le niveau de liaison du fibrinogène diminue lorsque le pH augmente. La liaison la plus élevée a été observée à pH 6,3. Les aptamères ne se sont pas liés au fibrinogène pour un pH supérieur à 6,8.

### Exemple 4 : préparation de supports d'affinité pour les aptamères identifiés

### 1. Matériel et méthode

### - Supports d'affinité

Deux supports d'affinité ont été préparés par greffage d'aptamères sur de la Sepharose NHS-activée (GE Healthcare). Le premier support d'affinité (support d'affinité N° 1) a été préparé par greffage d'aptamères de SEQ ID N° 66 (aptamère A5-1,9) comprenant un spacer en C6 avec un groupe amino terminal à son extrémité 5' et une désoxy-thymidine inversée à son extrémité 3'. Le second support d'affinité (support d'affinité N° 2) a été préparé par greffage d'aptamères de SEQ ID N° 67 (aptamère A5-2,9) comprenant un spacer en C6 avec un groupe amino terminal à son extrémité 5' et une désoxy-thymidine inversée à son extrémité 3'.

1 volume de gel de Sepharose NHS-activée placé dans une colonne a été rincé avec au moins 10 volumes de solution de HCl 0,1 M froide, puis équilibré avec au moins 8 volumes d'une solution d'acétate 100 mM froide, pH 4,0.

Après une centrifugation à 2000 g pendant 3 min, le surnageant est séparé et le gel égoutté est remis en suspension dans 2 volumes d'aptamère dans une solution d'acétate 100 mM pH 4,0. Cette suspension est incubée pendant 2 heures à la température ambiante sous agitation.

Ensuite, 1 volume de borate 200 mM pH 9 est ajouté, cette suspension est incubée à la température ambiante sous agitation pendant 2 h 30.

Après une centrifugation à 2000 g pendant 3 min, le surnageant est mis de côté. Le gel égoutté est remis en suspension dans 2 volumes de solution de Tris-HCl 0,1 M pH 8,5. La suspension est incubée à +4 °C sous agitation durant une nuit.

Après une incubation et une centrifugation à 2000 g pendant 3 min, le surnageant est mis de côté. Le gel est alternativement lavé avec 2 volumes d'acétate de sodium 0,1 M + NaCl 0,5 % pH 4,2 et 2 volumes de solution de Tris-HCl 0,1 M pH 8,5. Ce cycle de lavage est répété une fois.

Après une centrifugation à 2000 g pendant 3 min, le surnageant est séparé. Le gel égoutté est remis en suspension dans 2 volumes de tampon d'équilibrage.

| | | Support d'affinité N° 1 sur lequel sont greffés des groupements d'aptamères de SEQ ID N° 66 | Support d'affinité N° 2 sur lequel sont greffés des groupements d'aptamères de SEQ ID N° 67 |
|---|---|---|---|
| Purification du fibrinogène | Quantité d'aptamère utilisée pour le greffage | 4,2 mg | 2,5 mg |
| à partir du Plasma | Volume de gel greffé | 0,5 mL | 0,5 mL |
| Purification du fibrinogène | Quantité d'aptamère utilisée pour le greffage | 174 mg | 209 mg |
| à partir d'un produit fibrinogène semi-purifié | Volume de gel greffé | 24 mL | 42 mL |

### Exemple 5: Purification du fibrinogène à partir d'une solution de fibrinogène semi-purifiée sur le support d'affinité de l'exemple 4

### 1. Matériel et méthode

### - Conditions de la chromatographie d'affinité

Support d'affinité n° 1 : Une solution de fibrinogène semi-purifiée décongelée (IP1 : Produit intermédiaire de fibrinogène 1) obtenue à partir de plasma humain a été diluée 10 fois dans le tampon de liaison et le pH a été ajusté à 6,3. L'IP1 dilué a été soumis à des étapes de chromatographie sur le support n° 1. Cette étape a été répétée une fois pour obtenir une quantité suffisante de fibrinogène pour une étape d'ultrafiltration.

Support d'affinité n° 2 : Une solution de fibrinogène semi-purifiée décongelée (IP1 : Produit intermédiaire de fibrinogène 1) obtenue à partir de plasma humain a été diluée 10 fois dans le tampon de liaison et le pH a été ajusté à 6,3. L'IP1 dilué a été soumis à des étapes de chromatographie sur le support n° 2. Cette étape a été répétée une fois pour obtenir une quantité suffisante de fibrinogène pour une étape d'ultrafiltration.

Les conditions de la chromatographie d'affinité sont résumées pour chaque support d'affinité :

| | Support d'affinité N° 1 sur lequel sont greffés des groupements d'aptamères de SEQ ID N° 66 (A5-1.9) | Support d'affinité N° 2 sur lequel sont greffés des groupements d'aptamères de SEQ ID N° 67 (A5-2.9) |
|---|---|---|
| Tampon de liaison | MOPS 50 mM, MgCl₂ 5 mM, NaCl 150 mM, pH 6,3 | MOPS 50 mM, NaCl 150 mM, pH 6,3 |
| Tampon de lavage | Aucun | MOPS 50 mM, NaCl 2 M, pH 7,4 |
| Tampon d'élution | MOPS 50 mM, NaCl 150 mM, pH 7,4 | MOPS 50 mM, MgCl₂ 2 M, pH 7,4 |

Pour chaque support d'affinité, le fibrinogène a été élué dans des conditions modérées par modification de la composition du tampon. Pour les deux chromatographies sur les supports d'affinité N° 1 et n° 2, 2 fractions d'éluat ont été produites et regroupées pour une étape d'ultrafiltration.

### - Conditions de l'ultrafiltration

Pour chaque support d'affinité, un ensemble de fractions d'éluat a été soumis à une ultrafiltration à 100 kDa afin de concentrer le fibrinogène et de le formuler dans du citrate de sodium 10 mM, argiline à raison de 20 g/L à pH 7,4.

### - Procédés analytiques

| Protéines | Procédés de titrage |
|---|---|
| Fibronectine, Fibrinogène antigène | Néphélométrie |
| Facteur II, Facteur XI, Facteur XIII, Plasminogène | Elisa |
| Activité de coagulation du fibrinogène | Essai de coagulation (Procédé de von Clauss) |

### 2. Résultats

Les résultats sont présentés sur les figures 7A-7B et 7C-7D. Les figures 7A et 7B représentent le profil de chromatographie obtenu pour la purification du fibrinogène à partir d'une solution de fibrinogène semi-purifiée respectivement sur les supports d'affinité N° 1 et N° 2. Le fibrinogène a été éluée par élévation du pH à 7,4 et par addition de MgCl₂ pour le support d'affinité N° 2 et par élimination du Mg²⁺ pour le support d'affinité N° 1. L'analyse par électrophorèse des fractions obtenues par chromatographie (figures 7C et 7D) a montré que des contaminants présents dans le matériau chargé (IP1) sont notablement éliminés, pratiquement uniquement le fibrinogène étant visible dans l'éluat. De plus, des conditions réductrices montrent que le fibrinogène dans l'éluat est une forme native sans dégradation visible (Aα1 est la bande la plus importante parmi les bandes Aα).

Les rendements et la concentration de fibrinogène obtenus sont résumés dans le tableau ci-dessous :

| | Support d'affinité N° 1 | Support d'affinité N° 2 |
|---|---|---|
| Rendement de chromatographie (%) | 51 | 71 |
| Concentration du fibrinogène antigène obtenu après ultrafiltration (mg/ml) | 13,1 | 14,2 |

Le fibrinogène actif est mis en évidence par un rapport entre le fibrinogène coagulant et le fibrinogène antigène proche de 1. Une analyse du fibrinogène concentré et formulé préparé avec les deux supports d'affinité est détaillée dans le tableau suivant :

| | Activité de coagulation du fibrinogène g/L | rapport Fibrinogène de coagulation / antigène |
|---|---|---|
| Matériau de départ (fibrinogène IP1) | 17,6 | 1,17 |
| Concentré de fibrinogène purifié - Support n° 1 | 13,9 | 1,06 |
| Concentré de fibrinogène purifié - Support n° 2 | 14,5 | 1,02 |

Pour les deux fibrinogènes purifiés, le rapport entre le fibrinogène de coagulation et antigène était d'environ 1,0 pour les deux aptamères. Les conditions de chromatographie modérées ont permis la préparation du fibrinogène purifié ayant une activité conservée.

Le tableau ci-dessous montre le titrage des protéines contaminants dans le matériau de départ et dans la fraction de fibrinogène purifiée obtenue avec le support d'affinité de l'invention :

| | Fibrinogène semi-purifié (composition de départ) | Support | d'affinité n° 1 | Support | d'affinité n° 2 |
|---|---|---|---|---|---|
| Protéines Contaminantes | Concentration | Concentration | Elimination | Concentration | Elimination |
| Fibronectine | 0,55 g/L | 0,02 g/L | 96,3 % | 0,02 g/L | 95,1 % |
| Facteur II | 0,13 mUI/mL | 0,03 mUI/mL | 70,8 % | 0,04 mUI/mL | 66,3 % |
| Facteur XI | 21,0 mUI/mL | 2,8 mUI/mL | 83,8 % | 3,6 mUI/mL | 80,8 % |
| Facteur XIII | 10000 mUl/mL | 10 mUl/mL | 99,9 % | 42 mUl/mL | 99,5 % |
| Plasminogène | 56 pg/mL | 0,21 pg/mL | 99,5 % | 0,21 pg/mL | 99,6 % |

Une bonne élimination des protéines contaminantes est obtenue avec une élimination allant de 65 % jusqu'à 99 % du matériau de départ.

Les conditions de chromatographie ont permis l'élimination de plus de 99,5 % du plasminogène initial, qui est l'un des contaminants les plus problématiques du point de vue de la stabilité du fibrinogène.

Les aptamères identifiés par SELEX conviennent à une utilisation comme ligand d'affinité dans la purification de fibrinogène par chromatographie. Il convient de noter que les aptamères identifiés par le procédé de l'invention permettent la liaison sélective et ensuite l'élution du fibrinogène dans des conditions modérées et non dénaturantes.

### Exemple 6 : Purification de fibrinogène par chromatographie à partir de plasma

Support d'affinité N° 1 : Le plasma a été décongelé, filtré à 0,45 µm, dilué 10 fois avec du tampon de liaison et ensuite le pH a été ajusté à 6,3. Une solution diluée a été soumise à des étapes de chromatographie sur le support N° 1.

Support d'affinité N° 2 : Le plasma a été décongelé, filtré à 0,45 µm, dilué 10 fois avec du tampon de liaison et ensuite le pH a été ajusté à 6,3. Une solution diluée a été soumise à des étapes de chromatographie sur le support N° 2.

Les conditions de l'affinité sont résumées, pour chaque support d'affinité, dans le tableau ci-dessous :

| | Support d'affinité N° 1 sur lequel sont greffés des groupements d'aptamères de SEQ ID N° 66 (A5-1.9) | Support d'affinité N° 1 sur lequel sont greffés des groupements d'aptamères de SEQ ID N° 67 (A5-2.9) |
|---|---|---|
| Tampon de liaison | MOPS 50 mM, MgCl₂ 5 mM, NaCl 150 mM, pH 6,3 | MOPS 50 mM, NaCl 150 mM, pH 6,3 |
| Tampon de lavage | Retour à la ligne de base avec le tampon de liaison | MOPS 50 mM, NaCl 2 M, pH 7,4 |
| Tampon d'élution | MOPS 50 mM, NaCl 150 mM, pH 7,4 | MOPS 50 mM, MgCl₂ 2 M, pH 7,4 |
| Tampon de régénération | MOPS 50 mM, MgCl₂ 2 M, pH 7,4 | identique au tampon d'élution |

Pour chaque support d'affinité, le fibrinogène a été élué dans des conditions modérées par modification de la composition du tampon.

### 2. Résultats

Les résultats sont présentés sur les figures 5A-5B et 6A-6B. Les figures 5A et 6A représentent le profil de chromatographie obtenu pour la purification du fibrinogène à partir de plasma respectivement sur les supports d'affinité N° 1 et N° 2. Il convient de noter que la plupart des protéines contaminantes n'ont pas été retenues sur la phase stationnaire alors que le fibrinogène est lié au support. Le fibrinogène a été élué par élévation du pH à 7,4 et par addition de MgCl₂ 2 M pour le support d'affinité N° 2 et par élimination de Mg²⁺ pour le support d'affinité N° 1. L'analyse par électrophorèse des fractions obtenues par chromatographie (figure 5B et figure 6B) a montré que le fibrinogène était principalement présent dans la fraction d'élution alors que les protéines contaminantes étaient présentes dans la fraction non retenue, dans la fraction de lavage ou dans la fraction de régénération. En fait, les fractions d'élution ont migré sous forme d'une bande unique. La pureté relative (déterminée par SDS PAGE) des fractions de fibrinogène d'éluat était supérieure à 95 %.

Ces résultats mettent en évidence que les aptamères utilisés selon l'invention conviennent particulièrement à une utilisation comme ligands d'affinité dans la purification du fibrinogène à partir de compositions de départ complexes.

### Exemple 7: Optimisation de la séquence de base de SEQ ID N° : 66

### 1. Matériel et méthode

### - Préparation de variants de SEQ ID N° : 66 :

Dans le premier essai, 28 variants ont été conçus par élimination systématique de 2 nucléotides consécutifs par variant (1/2, 3/4, 5/6, etc.). Dans l'essai suivant, des combinaisons de délétions qui ne conduisaient pas à une perte d'affinité ont été combinées.

### - Essai de liaison compétitive

Afin de comparer l'affinité des variants conçus à celui de l'aptamère parental, un essai concurrent a été réalisé. Premièrement, une solution 1 µM de l'aptamère de SEQ ID N° 66 a été préparée à l'aide du tampon de liaison. La solution d'aptamère a été chauffée à 90 °C pendant 5 min, incubée dans de la glace pendant 5 min et équilibrée à la température ambiante pendant 10 min. La préparation a été injectée sur une puce de capteur revêtue de streptavidine SA de Biacore T200 instrument (GE Healthcare) à un débit de 10 µl/min pendant 7 min. Ensuite, des mélanges contenant un variant (2 µM) et le fibrinogène plasmatique humain (0,4 µM) ont été injectés sur l'aptamère immobilité à raison de 30 µl/min pendant 1 minute. La réponse obtenue pour les différents mélanges fragment/fibrinogène a été comparée.

### 2. Résultats

Comme le montre la figure 9, des variants de séquence de SEQ ID N° : 80-93 présentent une inhibition significative du signal de liaison et par conséquent ils possèdent une affinité considérable pour le fibrinogène. L'affinité la plus élevée a été observée pour les variants de SEQ ID N° : 80-87 contenant des délétions aux positions 01/02, aux positions 01/02/19/20/21, aux positions 01/02/14/15/16/20/21/22, aux positions 01/02/18/19/20/21, aux positions 01/02/18/19/20, aux positions 01/02/15/16/20/21, et aux positions 01/02/19/20, respectivement.

### Exemple 8 : stabilité d'une composition de fibrinogène liquide

### Composition de fibrinogène

La composition de fibrinogène a été obtenue à l'aide du support de chromatographie n°1 mettant en oeuvre l'aptamère A5-1.9 (SEQ ID N° 66) est formulée à minima avec de l'arginine 100 mM et du citrate 8,5 mM à pH 7,0.

### Stabilité de la composition de fibrinogène

La composition est mise en stabilité, sous air (à l'air libre), sans agitation, en chambre froide à +5°C.

Des échantillons sont prélevés avant la mise en stabilité (T0) et un mois après le début de la mise en stabilité (T1M).

### Résultats de la mise en stabilité à 5°C

Les résultats de cet essai de mise en stabilité sont présentés dans le tableau ci-dessous.

| Fibrinogène liquide (purifié par A5-1.9) Formulation minimale Mise en stabilité à 5°C | | **T0** | **T1M** |
|---|---|---|---|
| pH | | 6,9 | 7,0 |
| Osmolalité | | 206 | 204 |
| Fibrinogène coagulable | | 15,1 | 14,4 |
| Fibrinogène antigénique | | 14,2 | 13,4 |
| Ratio fibrinogène coagulable/antigènique | | 1,06 | 1,07 |
| SDS PAGE en conditions réduites | Aα1 (64 kDa) | 23,7 | 25,7 |
| | Aα2 (62 kDa) | 11,7 | 10,4 |
| | Aα3 (60 kDa) | 3,7 | 2,3 |
| | ΣAαₙ | 39,1 | 38,4 |
| | Bβ (54 kDa) | 31,2 | 31,9 |
| | γ' (50 kDa) | 6 | 5,6 |
| | γ (48 kDa) | 23,7 | 24,1 |
| | Σγ | 29,7 | 29,7 |

Les résultats après un mois de mise en stabilité montrent que :
- Les résultats de SDS PAGE en conditions réduites montrent une faible variation des profils des chaînes alpha, beta et gamma qui restent stables dans le temps, confirmant la conservation de l'intégrité du fibrinogène liquide ;
- Le ratio fibrinogène coagulable/fibrinogène antigène est conservé et stable à 1, confirmant la conservation de l'intégrité et de la fonctionnalité du fibrinogène liquide.

Les résultats de cet essai démontrent clairement que la composition de fibrinogène est stable sous forme liquide à 5°C pendant 1 mois.

**Tableau des séquences**

| **N° of SEQ ID** | **Description** |
|---|---|
| 1-57 | Aptamères du premier sous-groupe |
| 58-65 | Aptamères du second sous-groupe |
| 66 | Séquence de base de l'aptamère of SEQ ID N° : 1 (A.5.1.9) |
| 67 | Séquence de base de l'aptamère of SEQ ID N° : 58 (A.5.2.9) |
| 68-74 | Régions centrales de SEQ ID N° : 59-65 |
| 75 | Première séquence d'amorces |
| 76 | Seconde séquence d'amorces |
| 77-79 | Groupements de nucléotide présents dans la séquence consensus du second sous-groupe des aptamères |
| 80-93 | Variants de l'aptamère de SEQ ID N ° : 66 |
| 94 | Région centrale de SEQ ID N° : 1 |
| 95 | Région centrale de SEQ ID N° : 58 |

## Revendications

1. Composition comprenant du fibrinogène stable sous forme liquide.

2. Composition selon la revendication **1 caractérisée en ce qu'**elle est stable au moins 3 mois à 4°C.

3. Composition selon la revendication **1 ou 2 caractérisée en ce qu'**elle présente un ratio fibrinogène coagulable / fibrinogène antigène supérieur à 0,5; préférentiellement supérieur à 0,6; supérieur à 0,7; supérieur à 0,8; supérieur à 0,9; encore plus préférentiellement environ égal à 1,0.

4. Composition selon l'une quelconque des revendications **1 à 3 caractérisée en ce qu'**elle présente une quantité de monomères de fibrinogène conservés pendant la mise en test de stabilité supérieure à 50%, préférentiellement supérieure à 60%, préférentiellement supérieure à 70%, préférentiellement supérieure à 80%, préférentiellement supérieure à 90%, préférentiellement supérieure à 95% du taux initial de monomères de fibrinogène.

5. Composition selon l'une quelconque des revendications **1 à 4 caractérisée en ce qu'**elle présente une quantité de polymères formés pendant la mise en test de stabilité inférieure à 10%, préférentiellement inférieure à 20%, préférentiellement inférieure à 30%, préférentiellement inférieure à 40%, préférentiellement inférieure à 50% du taux initial de polymères de fibrinogène.

6. Composition selon l'une quelconque des revendications **1 à 5 caractérisée en ce que** :
- l'ensemble des chaînes alpha est conservée à au moins 50%, préférentiellement au moins 60%, préférentiellement au moins 70%, préférentiellement au moins 80%, préférentiellement au moins 90%; plus préférentiellement conservée à environ 100%, et/ou
- l'ensemble des chaînes beta est conservée à au moins 50%, préférentiellement au moins 60%, préférentiellement au moins 70%, préférentiellement au moins 80%, préférentiellement au moins 90%; plus préférentiellement conservée à environ 100%, et/ou
- l'ensemble des chaînes gamma est conservée à au moins 50%, préférentiellement au moins 60%, préférentiellement au moins 70%, préférentiellement au moins 80%, préférentiellement au moins 90%; plus préférentiellement conservée à environ 100%.

7. Composition selon l'une quelconque des revendications **1 à 6**, **caractérisée en ce que** la turbidité mesurée après le test de stabilité correspond à moins de 130%, moins de 120%, moins de 110% ; avantageusement correspond à 100% de la turbidité mesurée avant le test de stabilité.

8. Composition selon l'une quelconque des revendications **1 à 7**, **caractérisée en ce que** ladite composition est dépourvue de protéases et/ou d'activateurs de la fibrinolyse.

9. Composition selon l'une quelconque des revendications **1 à 8, caractérisée en ce que** ladite composition est dépourvue d'inhibiteurs de protéases et/ou d'anti-fibrinolytiques.

10. Composition selon l'une quelconque des revendications **1 à 9, caractérisée en ce que** ladite composition comprend en outre des excipients pharmaceutiquement acceptables.

11. Composition selon l'une quelconque des revendications **1 à 10, caractérisée en ce que** lesdits excipients pharmaceutiquement acceptables consistent en de l'arginine et du citrate.

12. Composition constituée de fibrinogène, d'arginine et de citrate et qui est stable sous forme liquide.

13. Composition selon l'une quelconque des revendications **1 à 12, caractérisée en ce qu'**elle est dépourvue d'isoleucine et/ou de glycine.

14. Composition selon l'une quelconque des revendications **1 à 13, caractérisée en ce qu'**elle est dépourvue d'albumine.

15. Composition selon l'une quelconque des revendications **1 à 14, caractérisée en ce qu'**elle est dépourvue d'ions métalliques, en particulier de calcium.

16. Composition comprenant du fibrinogène, ladite composition étant liquide et stable, et n'ayant subi aucune étape préalable de lyophilisation, dessiccation, déshydratation ou séchage.

17. Composition comprenant du fibrinogène, ladite composition étant liquide et stable et n'ayant subi aucune étape préalable de reconstitution d'un lyophilisat.

18. Composition comprenant du fibrinogène, ladite composition étant liquide et stable et susceptible d'être obtenue par le procédé comprenant les étapes suivantes :
- une étape de purification par chromatographie d'affinité,
- au moins une étape de sécurisation biologique, et
- une étape de formulation sous forme liquide.

19. Composition selon la revendication **18 caractérisée en ce que** la chromatographie d'affinité consiste en une chromatographie utilisant des ligands de type anticorps de lama ou aptamères.

20. Composition comprenant du fibrinogène liquide stable susceptible d'être obtenue selon le procédé comprenant les étapes suivantes :
- obtention d'un plasma sanguin ou d'une fraction de cryosurnageant de plasma sanguin,
- purification du plasma ou de la fraction de cryosurnageant de plasma sanguin par séparation sur gel de chromatographie d'affinité utilisant préférentiellement des ligands de type dérivés d'anticorps ou aptamères,
- récupération de la fraction adsorbée purifiée comprenant du fibrinogène,
- éventuellement ajout d'excipients pharmaceutiquement acceptables, de préférence de l'arginine et/ou du citrate.

21. Composition selon l'une quelconque des revendications **1 à 20** pour son utilisation en thérapie.

22. Composition pour son utilisation selon la revendication **21**, **caractérisée en ce que** ladite composition est administrée par voie intraveineuse.
